# FASCICULE DE BREVET EUROPEEN

(11) **EP 2 142 143 B1**
(45) Date de publication et mention de la délivrance du brevet: **12.11.2014**
(21) Numéro de dépôt: 08805533.0
(22) Date de dépôt: 30.04.2008
(51) Int. Cl.: A61F 2/24

(54) **VALVE CARDIAQUE PROTHÉTIQUE MÉCANIQUE**
MECHANISCHE PROTHETISCHE HERZKLAPPE
MECHANICAL PROSTHETIC HEART VALVE

(30) Priorité: 02.05.2007 FR 0703164
(43) Date de publication de la demande: 13.01.2010
(60) Demande divisionnaire: 10012015.3
(73) Titulaire: LAPEYRE INDUSTRIES LLC, Atlanta GA 30309 (US)
(72) Inventeur: LAPEYRE, Didier, F-27120 Chaignes (FR)
(74) Mandataire: Saam, Christophe
(86) Numéro de dépôt international: PCT/FR2008/000621
(87) Numéro de publication internationale: WO 2008/152224

(56) Documents cités:
- WO-A-92/16169
- US-A- 4 328 592
- US-A- 5 522 886
- US-A- 6 059 826
- US-A1- 2004 249 451
- US-B1- 6 395 024

## Description

La présente invention concerne une valve cardiaque prothétique mécanique.

De nos jours, environ 300 000 malades dans le monde bénéficient chaque année d'une prothèse valvulaire en remplacement d'une ou de plusieurs de leurs valves cardiaques détériorées soit par une maladie infectieuse soit par un processus dégénératif lié au vieillissement.

On distingue deux grandes familles de valves cardiaques prothétiques :
- les prothèses valvulaires d'origine biologique appelées bioprothèses, qui sont prélevées sur l'animal puis traitées chimiquement ou construites à partir de tissus biologiques sur le modèle d'une valve naturelle ;
- les prothèses valvulaires mécaniques qui sont des dispositifs sans rapport avec la forme d'une valve naturelle et fabriqués avec des matériaux artificiels résistant à l'usure et biologiquement compatibles.

Du fait de leur configuration anatomique et de leur mode de fonctionnement physiologique, les bioprothèses offrent des performances biologiques qui sont les mêmes que celles d'une valve cardiaque naturelle car elles respectent la structure naturelle de l'écoulement du sang à travers les cavités cardiaques et l'aorte.

Cette particularité des bioprothèses permet aux malades de faire l'économie d'un traitement anticoagulant toute leur vie durant, ce qui élimine le risque d'accidents hémorragiques consécutifs à la prise prolongée de ces médicaments et donc procure à ces malades une qualité de vie supérieure.

Ainsi, le patient peut oublier qu'il porte une valve cardiaque artificielle.

En outre, il convient de noter que les bioprothèses n'entraînent pas de nuisance acoustique, ce qui contribue également à faire oublier au patient qu'il est porteur d'une valve cardiaque artificielle.

Ces bioprothèses ont toutefois une durée de vie limitée en raison de leur calcification inéluctable avec le temps, ce qui impose un remplacement après une durée d'une dizaine d'années en moyenne. Une fois amorcée, cette calcification s'accélère et détruit la valve avec, pour conséquence, la dégradation progressive de la fonction valvulaire et ses répercussions sur le muscle cardiaque. Cette calcification survient plus rapidement chez les sujets jeunes que chez les sujets âgés, ce qui limite le champ d'application des bioprothèses aux sujets de plus de 65 ans ou aux sujets dont l'espérance de vie est inférieure à la durée de vie de la bioprothèse.

On notera que l'espérance de vie en France à 65 ans est de 17,7 années pour les hommes et de 21,7 années pour les femmes et que le remplacement d'une valve cardiaque déficiente est un acte de chirurgie lourde qui s'accompagne, au-delà de 75 ans, d'un taux de mortalité élevé. À ce risque s'ajoute, à cet âge, l'inconfort d'une opération chirurgicale majeure.

Contrairement aux bioprothèses, les dispositifs valvulaires artificiels de type mécanique ne sont pas dégradables et ont une durée de vie dépassant la durée de la vie humaine. En revanche, du fait de leur géométrie très éloignée du modèle naturel et de leur mode de fonctionnement non physiologique, ces valves mécaniques génèrent à chaque battement cardiaque des perturbations sur l'écoulement du sang sous forme de turbulences, de zones de recirculation, de tourbillons, de cisaillement des cellules sanguines et de ralentissement ou de stase du flux sur les parties du dispositif mécanique qui sont mal balayées par le flux sanguin, notamment les zones articulaires.

Ces perturbations de l'écoulement augmentent le temps de contact des cellules sanguines et l'intensité des réactions des protéines actives sur les matériaux prothétiques constituant ces dispositifs. Or, tout matériau étranger en contact avec le sang stimule par lui-même les processus de coagulation. Il résulte ainsi de l'interaction entre les perturbations de l'écoulement et les matériaux non biologiques :
- l'adhésion sur la surface de ces matériaux de protéines actives et de plaquettes sanguines,
- l'activation de la coagulation, et
- la formation sur ces surfaces de caillots organisés.

Ce phénomène biologique puissant est celui-là même qui gouverne le processus physiologique de cicatrisation de la paroi interne des vaisseaux. Il empêche les fuites du sang en dehors du système circulatoire. Il est donc indispensable au maintien de la vie et difficile à contrecarrer.

Cependant, les dépôts de coagulation peuvent non seulement entraver la fonction mécanique de la valve sur la circulation sanguine, ce qui met la vie des patients en danger, mais aussi migrer dans la circulation (embolies), le plus souvent dans la circulation cérébrale, et entraîner des troubles neurologiques souvent accompagnés de séquelles invalidantes.

À ces phénomènes de coagulation s'ajoute le traumatisme, répété à chaque cycle cardiaque sur les globules rouges, qui raccourcit leur durée de vie (hémolyse) et entraîne une réaction inflammatoire chronique de l'organisme tout entier. Cette réaction tend elle-même à augmenter la coagulabilité du sang, ce qui accroît la probabilité d'accidents de coagulation.

Ainsi, la thrombose génère la thrombose et crée une maladie chronique qui s'auto-entretient.

Pour remédier à cet inconvénient, tout malade porteur d'un dispositif valvulaire artificiel mécanique doit être protégé toute sa vie durant par un traitement anticoagulant avec le risque inhérent, soit d'accidents hémorragiques en cas de surdosage, soit d'accidents thromboemboliques en cas de sous-dosage.

Depuis le début des années soixantes, plusieurs générations de valves cardiaques mécaniques ont été successivement conçues pour réduire les perturbations que ces dispositifs génèrent sur l'écoulement, de façon à diminuer les risques de coagulation : d'abord, prothèses valvulaires constituées par une bille flottante dans une cage (STARR-EDWARD), puis, dans le début des années 70, prothèses de deuxième génération constituées par un disque basculant (BJÖRK-SHILEY) et, dix ans plus tard, prothèses de troisième génération à deux volets et ouverture latérale de type ST-JUDE MEDICAL. Cette troisième génération est aujourd'hui la plus utilisée et reprise sous des formes différentes par plusieurs fabricants.

Malgré ces améliorations, les valves de troisième génération restent traumatiques sur le sang et ne peuvent toujours pas fonctionner sur l'homme sans médicaments anticoagulants. En revanche, grâce à une expérience clinique de plus de 40 ans, le traitement anticoagulant est aujourd'hui bien codifié.

Les malades porteurs d'une valve mécanique en position aortique doivent maintenir leur coagulabilité sanguine (mesurée par une méthode biologique normalisée connue sous le terme "INR" pour "International Normalized Ratio") à un niveau au moins deux fois et demie supérieur à la valeur physiologique (INR 2,5).

Les malades porteurs d'une valve mécanique en position mitrale doivent maintenir leur coagulabilité sanguine à un taux au moins trois fois et demie supérieur à la valeur physiologique (INR 3,5).

Cette différence de "nocivité" des prothèses mécaniques entre la position aortique et la position mitrale est due au fait que la vitesse du sang est plus basse à travers l'orifice mitral qu'à travers l'orifice aortique. La durée de remplissage du coeur à travers la valve mitrale (typiquement de l'ordre de 450 millisecondes à 70 cycles par minutes) est en effet plus longue que la durée de l'éjection du sang à travers l'aorte (typiquement de l'ordre de 300 millisecondes). Le temps de contact du sang avec la valve prothétique en position mitrale est donc plus long, ce qui permet aux processus de coagulation d'aboutir à leur terme.

De plus, les valves mitrales étant de plus grandes dimensions, les surfaces de matériaux étrangers exposées aux dépôts biologiques sont plus étendues. Il est ainsi établi que le risque de complications thromboemboliques sur les malades porteurs de valves cardiaques mécaniques est deux fois plus élevé en position mitrale qu'en position aortique.

Sur les grandes séries de malades porteurs de valves cardiaques mécaniques, le taux moyen d'accidents de coagulation admis par la pratique médicale courante est statistiquement inférieur à 3 % par an et par malade, et le taux d'accidents hémorragiques est inférieur à 4% par an et par malade.

Ces données de l'état de l'art servent de référence aux cliniciens pour l'évaluation du potentiel thrombogénique d'une nouvelle valve cardiaque mécanique pendant les essais probatoires sur l'homme et sont déterminantes pour l'obtention des autorisations de sa mise sur le marché. Un taux de complications thromboemboliques ou d'hémorragies supérieur à 3-4 % entraînera le rejet du produit par la communauté médicale et le refus des agréments.

Pour autant que la protection anticoagulante soit correctement assurée, des millions de malades porteurs de valves cardiaques mécaniques dans le monde peuvent néanmoins vivre aujourd'hui dans des conditions acceptables. Ces malades qui étaient autrefois condamnés à mourir dans des délais courts peuvent, de nos jours, vivre de longues années. Toutefois leur espérance de vie, du fait du risque thromboembolique et hémorragique, reste notablement inférieure à celle des sujets de même âge non porteurs d'une valve cardiaque.

Le besoin impératif d'une protection anticoagulante pour tous les malades porteurs de valves cardiaques mécaniques se manifeste de façon particulièrement dramatique dans les pays où les structures médicales ne permettent pas un suivi satisfaisant du traitement anticoagulant. Dans ces pays, les maladies valvulaires sévissent à l'état endémique et touchent plus volontiers les sujets jeunes, les femmes et la position mitrale. Par exemple, plusieurs millions d'enfants de moins de 15 ans nécessitent en Inde un remplacement valvulaire prothétique. Ces sujets jeunes sont de mauvais candidats pour les valves de type biologique en raison des problèmes de calcification évoqués plus haut. Les valves cardiaques mécaniques sont donc plus volontiers employées mais s'accompagnent d'un taux de dysfonction par coagulation très supérieur à celui qui est observé dans les pays développés et ce risque majeur restreint leur utilisation. La thrombogénicité des valves cardiaques mécaniques représente dans ces pays un problème de santé publique et illustre le besoin de produits plus performants dont l'utilisation serait moins contraignante.

Il convient de noter que, même si le traitement anticoagulant est correctement suivi, le taux de complications reste préoccupant même dans les pays où les structures médicales sont adéquates. En effet, statistiquement, sur une période de 10 ans, un porteur de valve cardiaque mécanique sur deux aura subi une complication grave nécessitant son hospitalisation, soit du fait d'un accident de coagulation, soit du fait d'un accident hémorragique.

Les concepteurs de valves cardiaques mécaniques cherchent donc à améliorer les performances hydrodynamiques et le mode de fonctionnement de ces dispositifs pour réduire les perturbations qu'ils induisent sur l'écoulement du sang et, de ce fait, éliminer, ou au moins réduire les doses de médicaments anticoagulants nécessaires à la prévention de ces complications.

On connaît, d'après le brevet US 6 395 024, une valve cardiaque prothétique mécanique qui comporte une bague pourvue d'une surface périphérique intérieure centrée autour d'un axe et trois volets disposés à proximité de la surface périphérique intérieure de la bague. Ces trois volets sont adaptés à effectuer un mouvement de pivotement entre, d'une part, une position fermée empêchant le sang de s'écouler au travers de la valve et, d'autre part, une position ouverte dans laquelle l'écoulement de sang traverse la valve suivant une direction axiale.

La bague comporte, d'une part, un bord, appelé bord aval, reliant la surface périphérique intérieure à une surface périphérique extérieure et qui est placé du côté aval de l'écoulement et, d'autre part, trois créneaux ou protubérances qui s'étendent à partir de ce bord vers l'aval, suivant la direction axiale.

Chaque volet comporte une partie centrale pourvue de deux ailes latérales qui coopèrent chacune avec des moyens de guidage en rotation du volet respectivement aménagés sur les surfaces intérieures de deux créneaux consécutifs. L'espace dans lequel chaque aile latérale de volet pivote est appelé espace de pivotement.

En outre, deux fenêtres sont pratiquées de façon symétrique dans chacun des créneaux.

Chaque fenêtre permet un rinçage satisfaisant de la face externe des ailes latérales des volets par l'écoulement rétrograde.

Ainsi, lorsque la valve est implantée en position mitrale, cette face externe peut être balayée par l'écoulement du sang qui circule du ventricule vers l'aorte. Grâce à cette disposition, tout risque de dépôt biologique à cet endroit est donc éliminé.

De même, lorsque la valve est implantée en position aortique, le reflux du sang à travers ces fenêtres dans les sinus aortiques lorsque la valve est fermée peut assurer un rinçage de la face externe des ailes latérales, empêchant qu'un volume de sang puisse être retenu captif dans les espaces de pivotement du volet.

Pour parfaire cette protection contre la stagnation du sang dans les espaces de pivotement, un agencement supplémentaire a été aménagé : le bord inférieur des fenêtres décrites ci-dessus forme avec le bord d'attaque des ailes latérales des volets, lorsque ceux-ci sont en position ouverte, une deuxième ouverture ayant une forme de pertuis triangulaire. Cette deuxième ouverture (dénommée "cleft" en terminologie anglo-saxonne) est "dynamique" car la surface de l'orifice ainsi constitué augmente progressivement lorsque le volet passe de la position fermée à la position ouverte. Elle permet le passage direct vers l'extérieur des volets du sang véhiculé par l'écoulement antérograde et assure un balayage supplémentaire du bord d'attaque et de la face externe des ailes des volets.

Cependant, le Demandeur s'est aperçu à partir d'implantations effectuées sur l'animal que l'effet de cet agencement supplémentaire sur l'écoulement du sang n'était pas le même en position mitrale et en position aortique.

En effet, l'agencement susvisé s'est révélé efficace sur un grand nombre d'animaux implantés avec la valve en position mitrale et laissés pendant de nombreux mois sans protection anticoagulante, tandis qu'il en a été autrement des animaux sur lesquels la même valve a été implantée en position aortique.

En position mitrale, le sang sous faible pression peut s'écouler à travers les deuxièmes ouvertures ("clefts") de l'intérieur de la valve vers l'extérieur dans les espaces de pivotement des volets, pendant le remplissage ventriculaire, et effectuer un rinçage des espaces critiques de pivotement.

Cependant, la pression sanguine générée par le coeur, au cours de l'éjection ventriculaire, à travers la valve implantée en position aortique est dix fois supérieure à la pression sanguine qui s'exerce à travers la valve implantée en position mitrale.

Or, dans la mesure où les valves aortiques sont plus petites que les valves mitrales et où les "clefts" sont donc beaucoup plus étroits, l'effet de rinçage en position aortique crée, à chaque pulsation cardiaque, des "jets" latéraux puissants qui dépassent l'objectif de rinçage recherché et atteignent les valeurs traumatiques pour les cellules sanguines.

Le seuil traumatique reconnu par l'état de l'art en la matière se situe autour d'une force de 150 dynes/cm² pour les plaquettes du sang et de 1000 dynes/cm² pour les globules rouges. Au-delà de ces valeurs, les éléments du sang sont cisaillés, les plaquettes sanguines libèrent leurs agents coagulants, ce qui peut provoquer des complications de coagulation.

Ainsi, les "clefts" qui sont efficaces en position mitrale pour prévenir le ralentissement du sang dans les espaces de pivotement sont donc inutiles et potentiellement dangereux en position aortique.

L'expérience clinique a montré que les zones d'articulation d'une valve cardiaque mécanique constituent les zones les plus exposées aux phénomènes de coagulation.

Malheureusement, comme une valve cardiaque assure à chaque battement du coeur une fonction vitale sur la circulation du sang, les spécifications imposées par les impératifs de sécurité fonctionnelle sont prioritaires sur les problèmes de coagulation.

Ainsi, le mécanisme d'articulation des volets impose une géométrie peu favorable à une bonne structure du flux sanguin dans les espaces de pivotement. Il génère des cisaillements et des micro turbulences à proximité immédiate de surfaces relativement mal balayées par le courant sanguin.

L'amplitude de ce phénomène est liée au nombre de zones d'articulation de la valve. Elle est donc plus grande pour une valve cardiaque à trois volets qui comporte six espaces de pivotement que pour une valve cardiaque à deux volets qui n'en comporte que quatre.

De ce fait, les avantages de la valve cardiaque mécanique à trois volets en ce qui concerne la résistance aux complications de coagulation se trouvent grandement diminués si des dispositifs spécifiques ne sont pas mis en place.

Les malades qui ont besoin d'une valve cardiaque prothétique souhaitent n'être opérés qu'une seule fois et rester à l'abri des complications de coagulation qui peuvent survenir lorsque des corps étrangers sont présents dans le système circulatoire.

Malheureusement, pour éviter la formation de dépôts de coagulation, les malades sont obligés de prendre des médicaments anticoagulants durant toute leur vie, ce qui est contraignant et susceptible d'induire des complications hémorragiques du fait de la prise prolongée de tels médicaments.

Le document US 6,059,826 décrit une valve cardiaque à trois volets qui prennent une orientation parallèle ou presque parallèle à l'axe central en position d'ouverture complète mais ne sont pas parallèles à la surface périphérique interne.

Ce document montre que le volet prend appui, en position ouverte, contre la surface et la saillie uniquement par l'intermédiaire du bord latéral du volet et non pas l'intermédiaire de la surface extérieure de la portion terminale de l'aile latérale du volet. Par ailleurs, la surface extérieure du volet n'est pas convexe d'une portion terminale d'une aile latérale à la portion terminale de l'aile latérale opposée mais présente une convexité dans la partie centrale du volet et une concavité au niveau des portions terminales des ailes latérales.

Le document US 5,522,886 décrit une valve cardiaque comportant un support annulaire et au moins deux volets mobiles avec des dispositifs de charnière.

Il divulgue que chaque volet est en appui, en position ouverte, contre une portion de la surface interne d'une extension articulaire par l'intermédiaire de pions qui s'engagent dans des creux. Ainsi, le volet ne prend pas appui, en position ouverte, contre une portion de la surface interne de l'extension articulaire correspondante par l'intermédiaire d'une facette d'articulation.

Le document US2004/249451 décrit une valve cardiaque selon le préambule de la revendication 1.

La présente invention vise à remédier à au moins un des inconvénients de l'art antérieur en proposant une valve cardiaque prothétique mécanique, comprenant :
- un support annulaire comportant une surface périphérique interne centrée autour d'un axe longitudinal X,
- au moins deux volets mobiles qui sont agencés de façon articulée sur la surface périphérique interne du support de manière à pouvoir effectuer chacun un mouvement de rotation autour d'un axe de rotation de volet perpendiculaire à l'axe longitudinal, pour passer d'une position ouverte de la valve, dans laquelle les volets ouverts délimitent entre eux un orifice principal centré sur l'axe longitudinal et à travers lequel le sang s'écoule axialement, à une position fermée de la valve, dans laquelle les volets fermés empêchent le sang de refluer à travers l'orifice principal,
   le support annulaire comportant un bord placé du côté aval de l'écoulement antérograde, appelé bord aval, et plusieurs extensions articulaires qui s'étendent axialement à partir du bord aval et dont le nombre correspond à celui des volets, chaque volet comportant une partie centrale encadrée de façon symétrique par deux ailes latérales qui sont inclinées par rapport à cette partie centrale, ces deux ailes coopérant respectivement, pour permettre la rotation du volet, avec les surfaces internes de deux extensions articulaires par l'intermédiaire d'une portion dite terminale de chaque aile, chaque volet comportant à sa périphérie, d'une part, un bord d'attaque qui est disposé du côté amont de l'écoulement antérograde de sang et coopère avec la surface interne du support annulaire en position fermée du volet et d'autre part, un bord de fuite disposé du côté aval de l'écoulement antérograde, et qui comprend deux portions symétriques qui s'étendent respectivement depuis les portions terminales des ailes latérales jusqu'à une zone d'extrémité aval où elles se rejoignent pour former une pointe, chaque portion terminale de chaque aile latérale de chacun des volets comportant un bord latéral reliant le bord d'attaque et le bord de fuite, chaque volet comportant une surface extérieure tournée en position ouverte vers la surface périphérique interne du support annulaire et une surface intérieure tournée en position ouverte vers l'orifice principal de la valve et qui sont reliées l'une à l'autre par le bord d'attaque, le bord de fuite et les deux bords latéraux respectifs des deux portions terminales des deux ailes, la surface extérieure de chaque volet qui inclut la surface extérieure des ailes latérales ayant une forme générale convexe suivant une direction prise en se déplaçant d'une portion terminale d'une aile latérale à la portion terminale de l'aile latérale opposée, la surface intérieure ayant une forme générale concave suivant ladite direction, chaque portion terminale possédant une surface extérieure, appelée facette d'articulation, qui est une fraction de la surface extérieure du volet et qui vient en appui, lorsque le volet est ouvert, contre une portion de la surface interne de l'extension articulaire correspondante, appelée facette d'extension, les deux facettes d'articulation de chaque volet totalisant à elles deux une surface sensiblement inférieure à 5 % de la surface extérieure du volet.

En réduisant de façon drastique la surface extérieure de chaque aile latérale des volets en contact, en position ouverte, avec l'extension articulaire correspondante du support, on diminue considérablement la surface extérieure des volets qui n'est pas en contact direct avec l'écoulement de sang dans cette position.

De cette façon, que la valve soit implantée en position mitrale ou en positon aortique, la surface extérieure des volets est mieux balayée par l'écoulement de sang qu'auparavant, notamment, au droit des ailes latérales des volets.

La réduction considérable de la surface d'appui des volets en position ouverte supprime la nécessité d'aménager, au niveau des extensions articulaires, des ouvertures de rinçage, comme décrit dans le document de l'art antérieur précité, US 6 395 024.

Les échancrures pratiquées dans les extensions articulaires sur chacun de leurs côtés, voire à leur sommet, ont permis d'enlever une quantité significative de matériau réactif vis-à-vis du flux sanguin, ce qui améliore la résistance de la valve aux dépôts de coagulation selon l'invention et, plus généralement, ses performances fluidiques.

La diminution de la surface d'appui des volets n'est pas préjudiciable au fonctionnement de la valve dans la mesure où le Demandeur a remarqué qu'un large appui du volet sur le support de la valve à son ouverture n'était pas nécessaire, contrairement à ce qui se passe à la fermeture, où les forces hydrodynamiques exercées sur les butées d'appui ont une ampleur beaucoup plus grande.

En effet, en position ouverte l'effort exercé par l'écoulement sur le volet et donc sur la portion de la surface interne des extensions articulaires est minime.

Par ailleurs, l'invention permet de réduire considérablement le risque de blocage des volets à l'ouverture, qui pourrait survenir par interposition d'un dépôt de coagulation entre les surfaces extérieures des parties latérales des volets et les surfaces intérieures en regard des extensions articulaires correspondantes.

Si une telle interposition survenait, l'angle d'ouverture du ou des volets concernés serait réduit, ce qui aurait pour conséquence de créer une perturbation de l'écoulement susceptible de conduire à l'aggravation du phénomène et finalement à l'immobilisation du ou des volets en position fermée.

Outre la possibilité de thrombose articulaire entravant le fonctionnement du volet, cette interposition par des dépôts de coagulation peut également être la source d'embolies dans la circulation sanguine périphérique.

L'invention permet ainsi d'éliminer ou, à tout le moins, de réduire de façon drastique la prise de médicaments anticoagulants.

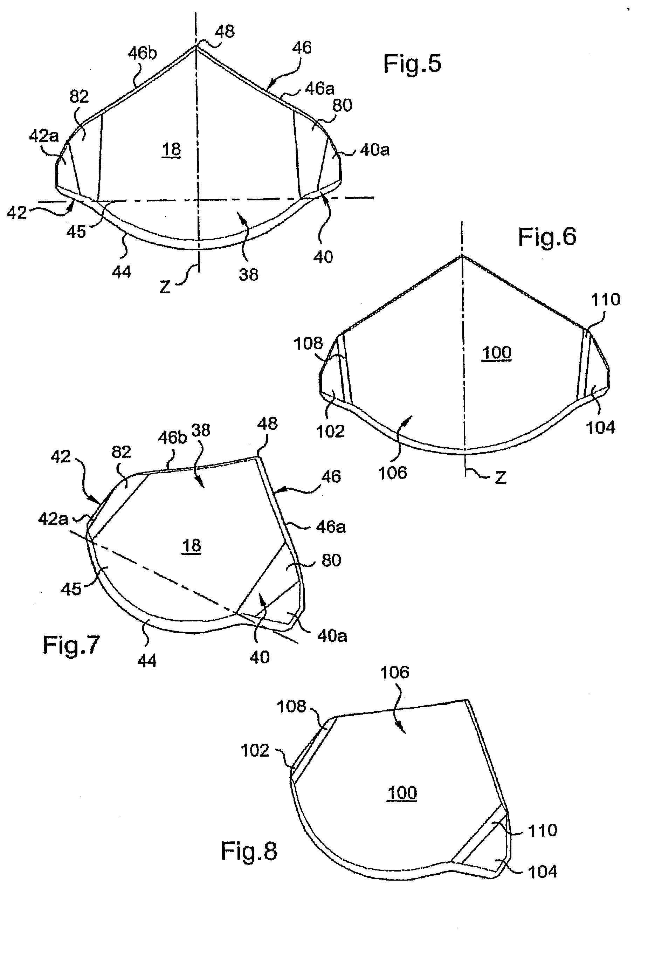

Sur les volets d'une valve cardiaque de l'art antérieur précité (tels que représentés sur les figures 6 et 8), la zone de jonction entre chaque aile latérale et la partie centrale du volet possède un faible rayon de courbure qui procure à cette zone la forme générale d'une arête.

Le Demandeur a pu constater, grâce à l'analyse de la microstructure de l'écoulement de sang à cet endroit, lorsque les volets sont en position ouverte, la présence en aval de la zone de jonction, à proximité des espaces de pivotement, d'un micro-courant tourbillonnaire qui se reproduit à chaque cycle.

Or, la turbulence du sang et l'augmentation à cet endroit du temps de résidence des globules rouges et des plaquettes favorise la formation et l'accrochage de caillots sanguins sur les surfaces immobiles avoisinantes.

Afin de supprimer cette perturbation locale de l'écoulement, il est prévu que chaque aile latérale de chacun des volets soit reliée à la partie centrale du volet par une zone de jonction dont la surface extérieure est convexe et qui, sur au moins une partie de sa longueur incluant la partie de la zone située vers l'aval de l'écoulement antérograde (bord de fuite), possède un rayon de courbure suffisamment grand pour éviter la formation d'écoulements tourbillonnaires au voisinage de cette surface.

Grâce à cet agencement, on réduit les distorsions localisées de l'écoulement au voisinage des espaces de pivotement des volets, l'écoulement suivant alors la surface extérieure des volets sans s'en détacher.

Par ailleurs, l'augmentation du rayon de courbure a pour conséquence de garder la partie du volet concernée par cette modification du rayon de courbure dans une zone d'écoulement soumise à un gradient de vitesses sensiblement proche de celui auquel est soumis le reste du volet, atténuant là encore davantage les distorsions de l'écoulement dans cette zone critique. La partie du volet concernée par cette disposition particulière est, par exemple, celle située à partir d'une distance d'environ 20 % du bord d'attaque du volet.

Un tel rayon de courbure dépend des dimensions du volet et est déterminé par l'homme du métier pour chaque taille de valve, de façon obtenir l'effet recherché.

Avec la configuration précitée, l'angle formé entre chaque aile latérale et la partie centrale de la face externe du volet se trouve augmenté par rapport à celui des volets de l'art antérieur.

Selon une caractéristique, le rayon de courbure de la zone de jonction située du côté aval de l'écoulement est d'au moins 2 mm pour une valve destinée à être implantée en position aortique et d'au moins 3 mm pour une valve destinée à être implantée en position mitrale.

Selon une autre caractéristique, chaque aile latérale de chacun des volets est reliée à la partie centrale du volet par une zone de jonction dont la surface extérieure est convexe et a la forme générale d'une portion de cône dont le sommet est situé vers l'amont de l'écoulement antérograde.

Selon cet agencement, le rayon de courbure entre chaque aile latérale et la partie centrale du volet n'est pas modifié de façon substantielle à proximité immédiate du bord d'attaque du volet mais il est d'autant plus modifié que l'on se rapproche du bord de fuite du volet (bord du volet situé du côté aval de l'écoulement).

Ainsi, une telle modification du rayon de courbure du volet dans la zone de jonction ne modifie pas le contour du bord d'attaque du volet, ni donc ses appuis sur la surface intérieure du support annulaire lors de la rotation du volet, de sa position ouverte à sa position fermée.

Selon une caractéristique, chaque aile latérale de chacun des volets est raccordée à la partie centrale du volet par une zone de jonction dont la surface extérieure est convexe et a la forme générale d'une portion de cylindre.

Selon une caractéristique, l'axe de rotation de chaque volet est virtuel, situé à l'extérieur du volet, entre ce dernier et le support annulaire, et s'étend suivant une direction prise en se déplaçant d'une aile latérale du volet à l'aile latérale opposée.

Selon une caractéristique, dans un plan perpendiculaire à l'axe longitudinal X de la valve, l'axe de rotation de volet est situé à une distance de l'axe longitudinal X qui est supérieure à 75 % du rayon du support annulaire.

Selon une caractéristique, chacune des facettes d'articulation d'un volet et la facette d'extension correspondante de l'extension articulaire concernée du support annulaire définissent entre elles, lorsque le volet est en position fermée, un espace de pivotement du volet, cet espace disparaissant lorsque la facette d'articulation du volet vient, en position ouverte, en appui contre la facette d'extension correspondante.

Selon une caractéristique, le volume de l'espace de pivotement est inférieur à 2/100e du volume déplacé par le volet lors de son passage de la position fermée à la position ouverte.

Selon une caractéristique, la surface extérieure de la partie centrale du volet a une forme générale sensiblement convexe suivant une direction prise en se déplaçant d'une aile latérale du volet à l'aile latérale opposée.

Selon une caractéristique, la partie centrale de chaque volet comporte une surface intérieure tournée vers l'orifice principal de la valve et qui a une forme générale sensiblement concave suivant une direction prise en se déplaçant d'une aile latérale du volet à l'aile latérale opposée.

Selon une caractéristique, lorsque la valve est en position ouverte, l'orifice principal délimité par les surfaces intérieures des volets présente, en projection dans un plan perpendiculaire à l'axe longitudinal du support annulaire, une section de passage offerte à l'écoulement qui est égale au moins à 75 % de la surface interne délimitée par le support annulaire dans ce même plan.

Selon une caractéristique, lorsque la valve est en position ouverte, chaque volet définit, entre sa surface extérieure et la portion de surface périphérique interne du support annulaire qui sépare les deux extensions articulaires avec lesquelles le volet coopère, un orifice secondaire.

Selon une caractéristique, chaque orifice secondaire a une forme générale de croissant de lune.

Selon une caractéristique, la dimension de l'orifice secondaire, prise suivant une direction radiale, en projection dans un plan perpendiculaire à l'axe longitudinal du support annulaire, est inférieure à 20 % du rayon interne du support annulaire.

Selon une caractéristique, chaque orifice secondaire présente, dans un plan perpendiculaire à l'axe longitudinal du support annulaire, une section de passage offerte à l'écoulement qui est inférieure à 7 % de la surface interne délimitée par le support annulaire dans ce même plan.

Selon une caractéristique, les extensions articulaires sont dépourvues chacune de toute ouverture traversante.

Selon une caractéristique, le support annulaire comporte sur sa surface périphérique interne, à proximité du bord aval et pour chaque volet, deux butées provoquant le pivotement immédiat du volet dans sa position ouverte lorsque la pression du sang s'exerce sur la face interne de ce volet.

Selon une caractéristique, le support annulaire comporte sur sa surface périphérique interne, pour chaque volet, deux moyens de soutien du volet en position fermée, lesdits moyens de soutien de chaque volet étant agencés entre les deux extensions articulaires avec lesquelles coopèrent respectivement les ailes latérales du volet.

Selon une caractéristique, en projection dans un plan perpendiculaire à l'axe longitudinal du support annulaire, chaque butée est espacée angulairement du moyen de soutien le plus proche d'une distance correspondant sensiblement à au moins la moitié de la largeur dudit moyen de soutien, la largeur étant mesurée, dans le plan considéré, suivant une direction tangentielle par rapport au support annulaire.

Selon une caractéristique, pour chaque volet, les butées sont agencées entre les moyens de soutien du volet.

Selon une caractéristique, chaque volet comporte à sa périphérie, d'une part, un bord d'attaque qui est disposé du côté amont de l'écoulement antérograde de sang et coopère avec la surface interne du support annulaire en position fermée du volet et, d'autre part, un bord de fuite disposé du côté aval de l'écoulement antérograde.

Selon une caractéristique, chaque moyen de soutien de volet coopère avec une zone de contact du bord d'attaque du volet suivant un contact surfacique et non pas linéaire lors de la fermeture dudit volet.

Selon une caractéristique, chaque moyen de soutien de volet présente une surface d'extrémité supérieure dont une portion située du côté opposé à l'extension articulaire la plus proche possède un rayon de courbure suffisamment grand pour coopérer avec la zone de contact rectiligne transversale du bord d'attaque du volet suivant un contact surfacique et non pas linéaire.

Selon une caractéristique, le bord de fuite de chaque volet a une forme sensiblement triangulaire et, en position fermée de la valve, les bords de fuite des trois volets coopèrent les uns avec les autres pour former un trièdre dont la pointe est dirigée vers l'aval.

Selon une caractéristique, chaque volet présente, dans sa partie centrale, au niveau du bord de fuite, une zone alignée suivant l'axe de symétrie du volet et qui est sensiblement en forme de spatule de ski à son extrémité libre aval, l'extrémité sensiblement spatulée du volet formant une pointe qui s'écarte du prolongement de la surface intérieure dudit volet d'un angle sensiblement compris entre 2 et 4°.

Selon une caractéristique, les trois extrémités sensiblement spatulées des volets restent distantes l'une de l'autre, en position fermée de la valve, d'au moins 50 microns et ménagent entre elles un interstice central en forme d'étoile à trois branches.

Selon une caractéristique, chacune des trois branches s'étend sur une distance correspondant au moins au tiers de la longueur totale du bord de fuite des volets.

Selon une caractéristique, chaque volet, d'une part, en position fermée, forme avec un plan perpendiculaire à l'axe longitudinal (X) du support annulaire un angle de fermeture compris entre 30° et 50° et, d'autre part, en position ouverte, est sensiblement parallèle à la direction de l'écoulement.

Selon une caractéristique, l'angle de fermeture est compris entre 40° et 50° pour les valves destinées à être implantées en position mitrale.

Selon une caractéristique, chaque volet présente sur sa surface extérieure une ou plusieurs zones pourvues de rainures qui favorisent l'orientation de l'écoulement sanguin vers les ailes latérales du volet.

Selon une caractéristique, le support annulaire comporte sur sa surface périphérique extérieure, pour les valves destinées à être implantées en position aortique, une nervure périphérique pour la fixation d'un anneau de suture, la nervure étant configurée de manière à ce que sa forme générale reproduise le profil d'une courbe sensiblement sinusoïdale ayant un sommet agencé au droit de chaque extension articulaire et un creux entre deux extensions articulaires consécutives.

Selon un autre aspect, l'invention a également pour objet un volet mobile destiné à être monté sur un support annulaire d'une valve cardiaque prothétique mécanique, comportant à sa périphérie, d'une part, un bord d'attaque qui est destiné à être disposé du côté amont de l'écoulement sanguin antérograde et, d'autre part, un bord de fuite qui est destiné à être disposé du côté aval de cet écoulement, le volet comprenant une partie centrale encadrée de façon symétrique par deux ailes latérales qui sont inclinées par rapport à cette partie centrale, chaque aile latérale étant reliée à la partie centrale par une zone de jonction dont la surface extérieure est convexe et qui, sur au moins une partie de sa longueur incluant le bord de fuite, possède un rayon de courbure suffisamment grand pour éviter le décollement du flux et la formation d'écoulements tourbillonnaires au voisinage de cette surface de jonction.

Selon une caractéristique, le rayon de courbure de la zone de jonction au droit du bord de fuite est d'au moins 2 mm pour une valve destinée à être implantée en position aortique et d'au moins 3 mm pour une valve destinée à être implantée en position mitrale.

Selon une caractéristique, la surface extérieure de la zone de jonction a la forme générale d'une portion de cône dont le sommet est situé du côté opposé au bord de fuite du volet.

Selon une caractéristique, la surface extérieure de la zone de jonction a la forme générale d'une portion de cylindre.

Selon une caractéristique, le volet comporte une surface extérieure et une surface intérieure opposées l'une de l'autre et reliant chacune le bord d'attaque au bord de fuite.

Selon une caractéristique, la surface extérieure de la partie centrale du volet a une forme générale sensiblement convexe suivant une direction prise en se déplaçant d'une aile latérale du volet à l'aile latérale opposée.

Selon une caractéristique, la surface intérieure de la partie centrale du volet a une forme générale sensiblement concave suivant une direction prise en se déplaçant d'une aile latérale du volet à l'aile latérale opposée.

Selon une caractéristique, le volet présente sur sa surface extérieure une ou plusieurs zones pourvues de rainures qui favorisent l'orientation de l'écoulement sanguin vers les ailes latérales.

Selon une caractéristique, le volet présente, dans sa partie centrale, au niveau du bord de fuite, une zone alignée suivant l'axe de symétrie du volet et qui est sensiblement en forme de spatule de ski à son extrémité libre, l'extrémité sensiblement spatulée du volet formant une pointe qui s'écarte du prolongement de la surface intérieure dudit volet d'un angle sensiblement compris entre 2 et 4°.

Selon une caractéristique, le volet est rigide.

Selon une caractéristique, le volet est réalisé à partir d'un matériau biocompatible et est réalisé au choix en carbone monolithique, en graphite avec un revêtement de carbone pyrolitique ou dans un polymère de synthèse doté de propriétés de résistance à l'usure comparables à celles du carbone pyrolitique.

D'autres caractéristiques et avantages apparaîtront au cours de la description qui va suivre, donnée uniquement à titre d'exemple non limitatif et faite en référence aux dessins annexés, sur lesquels :
- la figure 1 est une vue schématique en perspective d'une valve selon l'invention avec les volets disposés en position ouverte ;
- la figure 2 est une vue schématique en perspective de la valve de la figure 1 avec les volets en position fermée ;
- la figure 3 est une vue schématique partielle montrant la coopération d'un volet en position ouverte avec une extension articulaire selon l'invention et respectivement selon l'art antérieur (en pointillés), vus de l'extérieur de la valve ;
- la figure 4a est une vue schématique partielle en perspective de l'intérieur de la valve, montrant l'agencement d'un volet en position ouverte logé entre deux extensions articulaires du support ;
- la figure 4b est une vue schématique partielle agrandie d'un moyen de soutien coopérant avec le bord d'attaque d'un volet ;
- les figures 5 et 7 sont des vues schématiques de face et en perspective de la surface extérieure d'un volet selon l'invention ;
- les figures 6 et 8 sont des vues schématiques de face et en perspective de la surface extérieure d'un volet selon l'art antérieur ;
- la figure 9 est une vue d'un volet selon l'invention en section transversale dans un plan contenant l'axe de symétrie Z ;
- la figure 10 est une vue schématique de dessus d'une valve selon l'invention avec les volets en position fermée ;
- la figure 11 est une vue schématique partielle montrant l'agencement des ailes latérales de deux volets en position ouverte par rapport à une extension articulaire 32 de la valve ;
- la figure 12 est une vue schématique de dessus d'une valve selon l'invention avec les volets en position ouverte ;
- les figures 13 et 14 sont des vues schématiques partielles prises dans le plan de la partie centrale d'un volet selon l'invention, respectivement du bord d'attaque et du bord de fuite d'une des zones de jonction dudit volet ;
- la figure 15 est une vue schématique en coupe d'une section longitudinale d'un volet selon l'invention ;
- la figure 16 est une vue schématique partielle agrandie d'un espace de pivotement d'une valve selon l'invention ;
- la figure 17 est une vue schématique partielle montrant l'inclinaison d'un volet en position fermée d'une valve selon l'invention ;
- la figure 18 est une représentation schématique de l'écoulement de sang sur la surface extérieure d'un volet selon l'invention en l'absence de rainures ;
- la figure 19 est une vue schématique représentant l'écoulement de sang sur la surface extérieure d'un volet selon l'invention en présence de rainures ;
- la figure 20 est une vue schématique partielle d'une forme possible de rainures selon l'invention ;

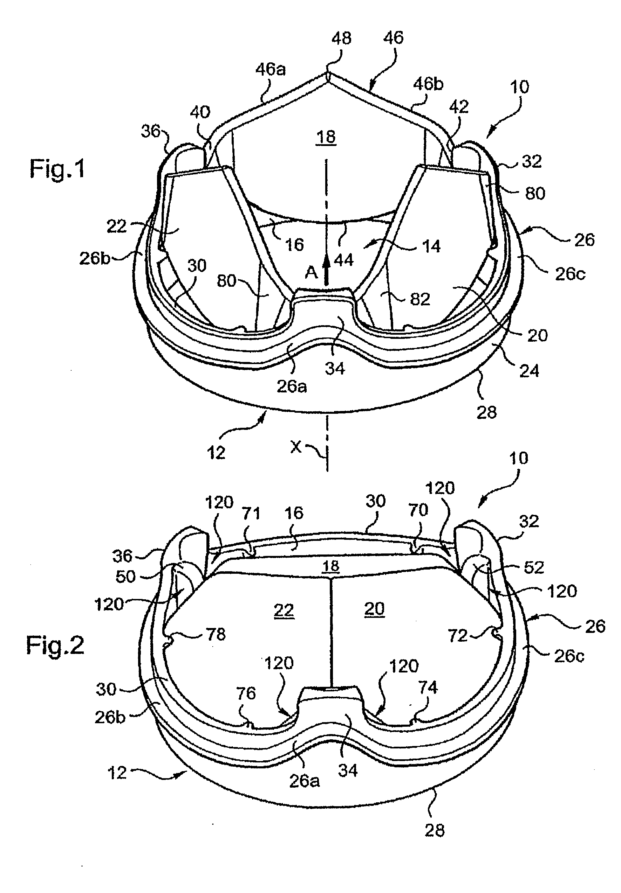

Comme représenté sur les figures 1 à 4b et désigné par la référence générale notée 10, une valve cardiaque prothétique mécanique selon l'invention comporte un support annulaire en forme de bague 12 qui définit à l'intérieur de celui-ci un passage interne 14 pour l'écoulement cyclique du sang sous l'action des contractions cardiaques.

L'écoulement traversant la valve 10 en position ouverte de celle-ci est qualifié de courant antérograde et son sens d'écoulement est indiqué par la flèche A sur la figure 1.

Par opposition, le courant circulant en sens inverse lors de la fermeture de la valve est qualifié de courant rétrograde.

Le passage interne central 14 pour l'écoulement de sang est délimité par la surface périphérique intérieure 16 du support annulaire 12 et qui va servir de support à trois volets mobiles 18, 20, 22 qui seront décrits ultérieurement.

Comme représenté sur les figures 1 et 2, la valve cardiaque 10 est centrée autour d'un axe longitudinal X et présente une symétrie de révolution autour de cet axe.

Le support annulaire 12 comporte également une surface périphérique extérieure 24 présentant une nervure périphérique 26 destinée à recevoir un anneau de suture non représenté, par exemple, en textile et qui permet au chirurgien de fixer de façon connue la valve aux tissus cardiaques par des points de suture.

Sur la figure 1 la valve est représentée en position ouverte dans laquelle les volets 18, 20 et 22 sont en position dite relevée ou ouverte, l'écoulement de sang traversant la valve dans le sens antérograde, tandis que sur la figure 2, la valve est représentée en position fermée avec les volets en position dite abaissée ou fermée.

On notera que la valve peut comporter, sans que cela n'affecte le principe de l'invention, seulement deux volets et, dans ce cas, le support annulaire 12 est de forme elliptique et les volets de forme ovale, ou plus de trois volets.

A cet égard, une valve conçue pour être implantée en position mitrale a, par exemple, deux volets de forme ovale mais elle peut également comporter trois volets d'une autre forme.

Le support annulaire 12 comporte un bord amont ou bord d'attaque 28 reliant la surface périphérique intérieure 16 à la surface périphérique extérieure 24 et qui est placé du côté amont de l'écoulement antérograde.

Le support annulaire comporte également un bord aval ou bord de fuite 30 qui est placé du côté aval de l'écoulement antérograde et qui relie lui aussi la surface périphérique intérieure 16 à la surface périphérique extérieure 24 du support annulaire.

Le support 12 comporte également trois extensions ou protubérances articulaires 32, 34, 36 qui s'étendent à partir du bord aval 30 vers l'aval, parallèlement à la direction d'axe longitudinal X, et qui forment ainsi des créneaux s'étendant axialement par rapport au bord périphérique 30 et dont la base est sensiblement de même largeur (dimension perpendiculaire à l'axe X) que le sommet.

Ces extensions logent les zones d'articulation avec lesquelles les volets mobiles coopèrent pour passer de la position ouverte à la position fermée et inversement.

On notera d'ailleurs que la largeur des extensions articulaires à leur sommet est sensiblement égale à la largeur des zones articulaires.

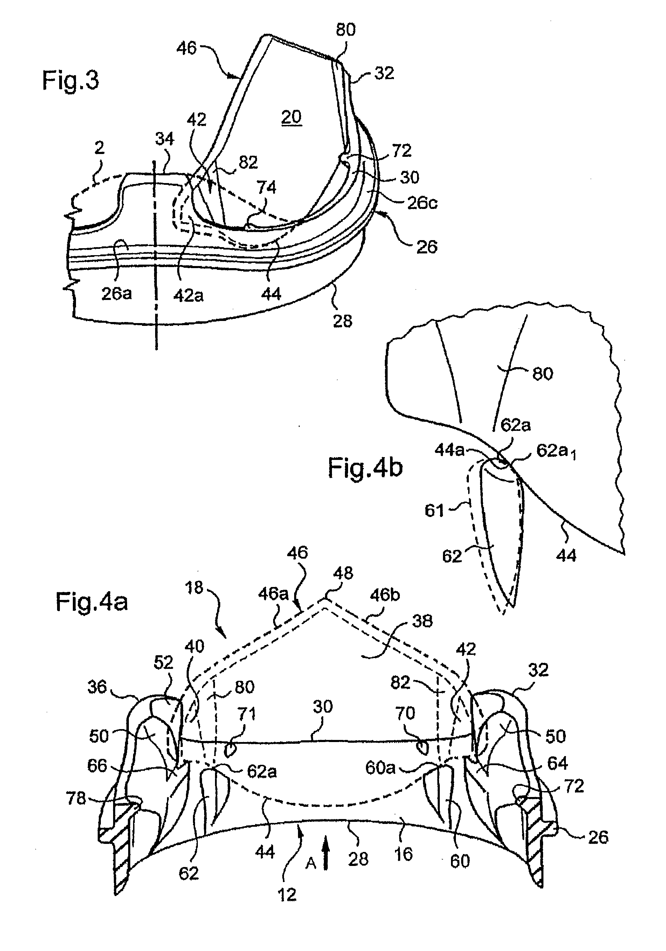

Ces extensions articulaires 32, 34, 36, en nombre égal à celui des volets, sont en effet de dimensions réduites par rapport aux créneaux équipant des valves cardiaques de l'art antérieur, comme représenté de façon schématique sur la vue partielle de la figure 3, où l'on a volontairement superposé une extension articulaire en pointillés notée 2 d'une valve cardiaque de l'art antérieur à l'extension articulaire 34 de la valve 10 selon l'invention.

Pour passer de l'ancienne configuration de l'extension articulaire 2 à la nouvelle configuration de l'extension 34, la surface de l'extension articulaire 2 en projection dans le plan de la figure 3 a été réduite d'au moins 50 %.

Comme représenté sur les figures 1 à 4b, les extensions articulaires de la valve 10 selon l'invention ne comportent aucune ouverture traversante, contrairement aux extensions articulaires des valves de l'art antérieur et notamment celles exposées dans le brevet US 6 395 024.

Le fait que les extensions articulaires soient dépourvues d'ouverture traversante améliore le comportement de la valve selon l'invention vis-à-vis de l'écoulement, lorsque celle-ci est implantée en position aortique.

En effet, dans une telle position, la valve exposée dans le brevet US 6 395 024 présente six petites ouvertures réparties deux par deux symétriquement sur chacune des extensions articulaires et dont la fonction est de permettre le nettoyage du bord d'attaque des volets lorsque ceux-ci sont en position ouverte (relevée).

Etant donné qu'en position aortique le régime de l'écoulement sanguin est un régime de hautes pressions, il se produit un phénomène de cisaillement de l'écoulement sanguin à travers ces petites ouvertures. Ceci aboutit à la création de six jets latéraux à vitesses élevées contre la paroi aortique et il en résulte une activation du phénomène de la coagulation.

La conséquence directe de l'enchaînement de ces évènements est la formation locale d'un caillot limitant progressivement le débattement des volets, risquant ainsi d'entraîner un dysfonctionnement de la valve et une insuffisance circulatoire pouvant conduire au décès du patient.

L'absence d'ouverture traversante dans les extensions articulaires permet d'éviter ce risque.

La description qui va suivre du volet 18 représenté sur les figures 1, 4a, 4b, 5 et 7 est identique pour tous les autres volets équipant la valve 10 selon l'invention.

Le volet 18 comporte une partie centrale 38 à laquelle sont raccordées deux ailes latérales 40, 42 encadrant cette dernière de façon symétrique et qui sont inclinées par rapport à elle (figures 1 et 7).

Le volet 18 est symétrique par rapport à un plan passant par l'axe Z (axe de symétrie) et qui est perpendiculaire au plan de la figure 5.

Le volet 18 comporte un bord d'attaque 44 qui, en position ouverte du volet, telle que représentée sur les figures 1, 4a et 4b, est placé du côté amont de l'écoulement antérograde (flèche A) et, en position fermée, coopère avec la surface périphérique intérieure 16 du support annulaire 12 avec des moyens spécifiques aménagés sur cette surface, comme on le verra ultérieurement.

Ce bord d'attaque 44 présente une forme convexe dont la courbure orientée vers le bas (figures 4a, 4b, 5 et 7) est adaptée à coopérer avec la surface intérieure 16 de la valve.

Par ailleurs, le volet 18 comporte, sur le côté du volet opposé au côté où est situé le bord d'attaque, un bord de fuite 46 qui est disposé du côté aval de l'écoulement antérograde.

Comme représenté sur les figures 1, 4a, 5 et 7, le bord de fuite 46 comprend deux portions symétriques 46a et 46b qui s'étendent respectivement depuis les ailes latérales 40 et 42 jusqu'à une zone d'extrémité aval 48 où elles se rejoignent pour former une pointe.

Cette pointe 48 est alignée suivant l'axe de symétrie Z du volet.

Les portions 46a et 46b confèrent ainsi au bord de fuite 46 une forme sensiblement triangulaire de V renversé dont la pointe coïncide avec la zone d'extrémité 48.

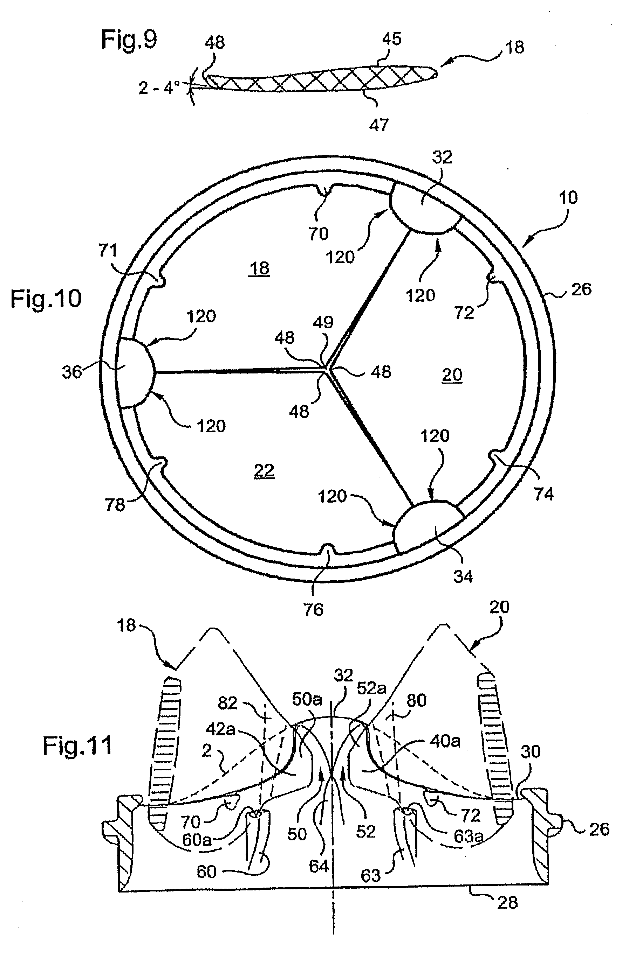

En position fermée de la valve (figures 2 et 10), les bords de fuite des trois volets coopèrent les uns avec les autres pour former un trièdre dont la pointe est dirigée vers l'aval.

La zone d'extrémité 48 qui est visible sur la figure 7 montrant la surface extérieure 45 du volet 18 est, par exemple, relevée par rapport à la surface extérieure du volet de manière à adopter la forme sensiblement "spatulée" d'une extrémité d'une spatule de ski.

On notera à cet égard que cette surface extérieure a, par exemple, une forme générale plane suivant une direction prise en se déplaçant d'une aile latérale du volet à l'aile latérale opposée.

Plus particulièrement, comme représenté sur la figure 9, l'extrémité sensiblement spatulée 48 du volet forme une pointe qui s'écarte du prolongement de la surface intérieure 47 du volet d'un angle qui est sensiblement compris entre 2° et 4°.

Ainsi, lorsque le volet est placé dans l'écoulement, l'extrémité spatulée 48 du volet n'est pas parallèle à l'écoulement alors que le corps du volet est sensiblement parallèle à la direction de l'écoulement.

La présence de l'extrémité libre relevée 48 de chaque volet renforce le mécanisme hydrodynamique de fermeture anticipée du volet accompagnant la décélération de l'écoulement antérograde et qui est dû à l'établissement progressif pendant cette phase d'un subtil gradient transitoire positif de pression entre les surfaces externe et interne du volet.

La figure 10 illustre, en vue de dessus, les volets 18, 20, 22 de la valve 10 en position fermée, dans laquelle les extrémités spatulées 48 sont écartées l'une de l'autre d'une distance au moins égale à 50 microns. Un interstice central 49 en forme d'étoile à trois branches est ainsi ménagé entre les bords de fuite respectifs de ces volets.

Cet interstice empêche tout risque de cavitation à la fermeture des volets et évite la génération de bruit à la fermeture en éliminant le contact entre les bords de fuite des volets au niveau de leurs zones d'extrémité 48.

En outre, si un peu d'usure du bord d'attaque des volets apparaît à long terme au niveau de leur surface de contact avec la surface intérieure du support annulaire, les volets s'abaisseront sensiblement au dessous de l'angle de fermeture nominal mais un interstice sera malgré tout toujours présent pour éviter le contact entre les zones d'extrémité 48 des bords de fuite des volets.

On notera que chacune des branches s'étend sur une distance correspondant au moins au tiers de la longueur totale du bord de fuite des volets.

Comme représenté sur les figures 1, 2 et 4a, 4b, le volet 18, comme tous les autres volets, et notamment le volet 20 sur les figures 1 à 3, coopère avec la surface périphérique intérieure 16 du support annulaire 12 et, plus spécifiquement, avec des moyens de guidage en rotation du volet, ainsi qu'avec des moyens d'appui qui sont aménagés radialement à la surface périphérique intérieure de la valve.

Ainsi articulés sur la surface périphérique interne 16, les volets sont aptes à effectuer un mouvement de rotation entre leur position ouverte de la figure 1 et leur position fermée de la figure 2.

Les moyens de guidage en rotation du volet comprennent deux évidements profilés 50 et 52 aménagés dans l'épaisseur des deux extensions articulaires respectives 32 et 36 et qui forment des pistes ou arcs de guidage et de retenue des ailes latérales du volet. Plus particulièrement, ces pistes ou arcs coopèrent avec des parties du bord de fuite 46 du volet qui sont situées au niveau d'une portion dite terminale des ailes latérales 40, 42 (figures 3, 4a et 11).

Les arcs de guidage (figure 11) agencés symétriquement sur la surface périphérique interne de chaque extension articulaire sont décrits de façon plus détaillée dans le brevet français 2 642 960 auquel on pourra se reporter.

La valve 10 comporte également plusieurs moyens d'appui différents de chaque volet qui sont aménagés sur la surface périphérique intérieure 16 du support 12.

En particulier, deux premiers moyens d'appui ou de soutien inférieurs 60, 62 du volet 18 (figures 4a et 4b) présentent une forme hydrodynamique profilée dont la section transversale augmente dans le sens d'écoulement du flux antérograde La forme profilée se termine par une surface d'extrémité supérieure 60a, 62a en forme d'arceau asymétrique dont la pente est plus inclinée du côté opposé aux extensions articulaires comme le montre la figure 4b pour le moyen de soutien 62.

La surface d'extrémité supérieure 62a coopère avec une zone de contact 44a du bord d'attaque 44 afin d'établir un contact surfacique entre eux lors de la fermeture du volet, lorsque ladite zone de contact se déplace en direction de la base d'insertion du moyen de soutien qui est localisée sur la surface périphérique interne 16 de la valve.

Ce contact surfacique permet de distribuer l'usure due au contact des deux éléments (bord d'attaque du volet et moyen de soutien) sur une surface au lieu d'avoir un contact suivant une ligne de contact, comme ce serait le cas avec le profil symétrique du moyen de soutien 61 représenté en pointillés sur la figure 4b. La répartition des forces est donc mieux répartie grâce au profil asymétrique de la tête (extrémité supérieure) du moyen de soutien 62 et, plus particulièrement, grâce à la portion 62a1 de la tête de ce dernier qui possède un rayon de courbure suffisamment grand pour obtenir un contact surfacique avec la zone de contact rectiligne 44a du bord d'attaque. La portion 62a1 présente une forme sensiblement plane, par exemple, réalisée sous la forme d'un méplat, conférant ainsi à la surface d'extrémité supérieure 62a un profil convexe du côté de l'extension articulaire la plus proche et sensiblement plat du côté opposé.

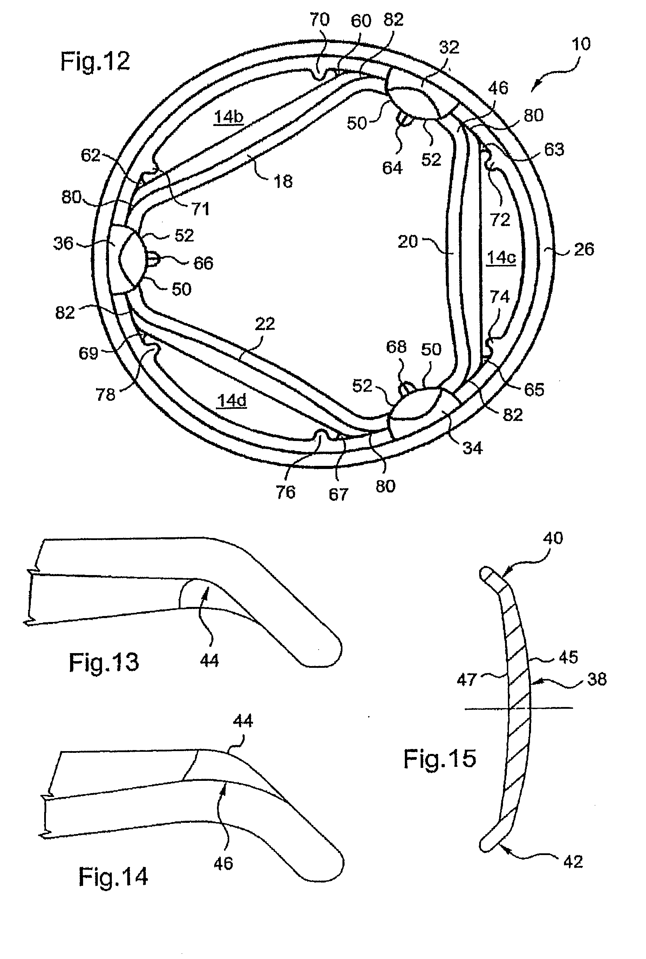

En position fermée le volet 18 repose ensuite par son bord d'attaque 44 (figure 4a) sur les surfaces d'extrémité supérieure 60a, 62a des moyens de soutien et, plus particulièrement, sur les portions aplanies de ces surfaces. De façon identique, deux premiers moyens d'appui inférieurs distincts du même type que celui décrit ci-dessus sont également prévus sur la valve pour chaque autre volet : les moyens d'appui 63, 65 pour le volet 20 et les moyens d'appui 67, 69 pour le volet 22, comme représenté sur la figure 12.

La valve comporte également des seconds moyens d'appui ou de soutien inférieurs agencés sensiblement dans la partie médiane et inférieure de chaque extension articulaire (figures 4a, 11 et 12) et qui se présentent sous la forme d'un élément 64, 66, 68 en forme de proue de navire pointée vers le haut, profilée dans la direction de l'écoulement antérograde. Chacun des éléments profilés 64, 66, 68 des extensions articulaires respectives 32, 36 et 34 comporte des bords latéraux suffisamment espacés (environ d'une distance égale à l'épaisseur des volets) pour servir d'appui aux bords latéraux des volets en position fermée.

Par ailleurs, des moyens d'appui dits supérieurs du volet 18, notés 70, 71 pour le volet 18 (respectivement 72, 74 et 76, 78 pour les volets 20 et 22), sont agencés au niveau du bord aval 30 du support annulaire de façon décalée axialement suivant l'axe longitudinal X par rapport aux premiers moyens d'appui inférieurs (figures 4a et 11).

En outre, comme représenté sur les figures 11 et 12, les premiers moyens d'appui inférieurs 60 et 63 et les moyens d'appui supérieurs respectifs 70 et 72 de ces volets sont décalés radialement les uns par rapport aux autres afin d'éviter que les moyens d'appui supérieurs soient placés dans le sillage des premiers moyens d'appui inférieurs. Ceci permet ainsi d'éviter la création, entre ces moyens d'appui inférieurs et supérieurs, de micro-perturbations de l'écoulement qui seraient favorables à l'activation des plaquettes du sang.

Cet agencement assure également que les surfaces du volet et du support situées entre les premiers moyens d'appui inférieurs et les moyens d'appui supérieurs sont suffisamment balayées par l'écoulement au cours du cycle cardiaque. Notamment, la surface d'extrémité supérieure de chaque premier moyen de soutien inférieur est bien exposée au courant rétrograde pendant la fermeture du volet.

Les moyens d'appui supérieurs 70 et 71 du volet 18 agencés entre les deux extensions articulaires 32, 36 (figure 4a) avec lesquelles coopèrent respectivement les ailes latérales de ce volet jouent le rôle de butées supérieures lors du mouvement d'ouverture du volet. Ces butées provoquent ainsi le pivotement du volet autour de son axe de rotation qui sera décrit ultérieurement, lorsque la pression de l'écoulement de sang s'exerce sur la surface interne de ce volet.

Plus particulièrement, les butées supérieures 70 et 71 entrent en contact avec la surface externe du volet dans sa partie amont dès les premières millisecondes d'ouverture de la valve.

En effet, lorsque la pression sanguine s'exerce sur la surface intérieure du volet fermé et le soulève de quelques dixièmes de millimètre (ceci est rendu possible par le jeu aménagé entre la partie inférieure des butées et la surface supérieure extérieure du volet quand celui-ci repose sur les surfaces d'extrémité 60a, 62a des premiers moyens d'appui inférieurs), le contact du volet avec ces butées provoque le pivotement symétrique de ses deux ailes latérales autour de l'axe de rotation et le relèvement du volet. En raison de ce pivotement quasi instantané, la surface extérieure du volet s'écarte des butées, ménageant ainsi entre ces butées et cette surface du volet un large passage pour le flux sanguin.

On notera par ailleurs qu'en position ouverte les volets ne reposent pas sur les moyens d'appui inférieurs, ceux-ci ne servant de support que lors de la fermeture des volets.

De plus, en positionnant les moyens d'appui supérieurs 70 et 71 entre les premiers moyens d'appui inférieurs 60 et 62, on augmente sensiblement le volume des moyens d'appui supérieurs, rendant ainsi plus grande la surface d'impact entre ces derniers et la surface extérieure du volet à proximité de son bord d'attaque. De ce fait, on réduit la concentration de contraintes mécaniques au niveau du point de contact, ce qui évite à long terme les altérations possibles de l'état de surface local du volet.

Il convient toutefois de ne pas trop éloigner les moyens d'appui supérieurs des premiers moyens d'appui inférieurs pour conserver l'effet d'ouverture synchrone et symétrique des deux ailes latérales du volet et ne pas augmenter le volume des moyens d'appui supérieurs dans une proportion qui risquerait d'induire une perturbation inutile sur l'écoulement sanguin.

Pour ces raisons, dans l'exemple de réalisation décrit ici, il est prévu de décaler radialement ou angulairement (en projection dans un plan perpendiculaire à l'axe X) chaque moyen d'appui supérieur 70, 71 de son premier moyen d'appui inférieur le plus proche 60, 62 d'une distance qui correspond sensiblement à au moins une fois la dimension (largeur) du premier moyen d'appui inférieur qui est mesurée radialement.

Par exemple, pour une valve cardiaque de diamètre extérieur égal à 29 mm, la dimension ou largeur radiale du moyen d'appui inférieur est d'environ 1,5 mm, et le moyen d'appui supérieur est donc espacé radialement d'au moins 1,5 mm du sillage du premier moyen d'appui inférieur correspondant.

Le moyen d'appui supérieur (butée) est de préférence plus large dans sa partie amont et plus effilé dans sa partie aval puisque seule la partie amont rentre en contact avec la surface extérieure du volet à son ouverture et qu'il importe de réduire localement la concentration de contraintes lors de l'impact.

Comme représenté sur la figure 4a, le bord d'attaque 44 du volet 18 est agencé entre les premiers moyens d'appui inférieurs 60, 62 et les moyens d'appui supérieurs 70, 71.

On notera que les moyens de guidage en rotation de chaque volet définissent un axe de rotation (représenté en traits mixtes sur les figures 5 et 7) qui s'étend dans une direction prise en se déplaçant d'une aile latérale du volet à l'aile latérale opposée. L'axe de rotation est situé à une distance de l'axe longitudinal X de la valve (dans un plan perpendiculaire à cet axe) qui est supérieure à 75 % du rayon du support annulaire 12 d'un volet, et ce, tout en permettant l'écoulement sanguin entre la surface extérieure du volet et la surface périphérique intérieure 16 du support annulaire.

Par ailleurs, chaque axe de rotation est virtuel car il est situé entièrement à l'extérieur du volet correspondant, entre ce dernier et le support annulaire. L'axe est donc très excentré par rapport au centre de gravité du volet. Ainsi, la résultante des forces de frottement agissant sur le volet exerce par rapport à l'axe virtuel un mouvement suffisant pour amorcer la fermeture du volet lors de la décélération de l'écoulement sanguin. Ceci favorise le mouvement de fermeture et le rend beaucoup moins violent qu'avec certaines valves de l'art antérieur dont les volets se ferment brutalement, occasionnant à la fois du bruit et un traumatisme pour les cellules sanguines circulantes.

Cette disposition excentrée des axes de rotation des volets permet de disposer les volets, en position d'ouverture de la valve, sensiblement parallèlement à l'axe de l'écoulement sanguin, voire même dans un plan dépassant sensiblement l'angle de 90° par rapport au plan perpendiculaire à l'axe X, car les seules forces de frottement sont suffisantes pour amorcer leur fermeture.

Comme déjà relevé plus haut, la présence de l'extrémité relevée en forme de spatule de ski de la zone d'extrémité 48 de chaque volet contribue à favoriser la fermeture précoce des volets lors de la décélération de l'écoulement en utilisant les forces naturelles de l'écoulement.

En outre, en éloignant les moyens d'appui supérieurs 70, 71 des premiers moyens d'appui inférieurs 60, 62 du volet 18, les moyens d'appui supérieurs s'éloignent de l'axe de rotation du volet et augmentent ainsi l'effet de levier recherché lorsque le bord supérieur du volet se soulève du fait de la pression exercée sur sa surface intérieure en début de phase d'ouverture du cycle cardiaque.

Une très faible force hydrodynamique appliquée sur la surface interne du volet fermé provoque alors de façon quasi-immédiate le basculement symétrique du volet autour de son axe de rotation.

Comme déjà décrit plus haut en référence à la figure 4a, les extensions articulaires agencées sur le bord aval du support annulaire 12 sont de dimensions considérablement réduites par rapport aux extensions articulaires des valves à trois volets de l'art antérieur.

De ce fait, lorsque les volets sont relevés (valve en position ouverte comme sur les figures 1, 3, 4a, 11 et 12) la surface extérieure de chaque aile latérale de chacun des volets qui est disposée en appui contre une partie latérale d'une extension articulaire correspondante est considérablement réduite par rapport à l'art antérieur. En effet, comme représenté sur les figures 3 et 11, seule une fraction de la surface extérieure de chaque aile latérale est en contact avec une partie de l'extension articulaire, alors que, selon l'art antérieur, la quasi-totalité de la surface extérieure de chaque aile latérale du volet 20 est agencée contre une partie beaucoup plus large de l'extension articulaire correspondante 2 (en pointillés).

Ainsi, pour l'aile latérale 42 du volet 20 de la figure 3, seule la surface extérieure de la portion terminale 42a, appelée facette d'articulation, de cette aile latérale 42 est en vis-à-vis et en appui contre une portion de la surface interne de l'extension articulaire 34, appelée facette d'extension.

Sur la figure 11, on a représenté en traits interrompus les facettes d'articulation 42a et 40a des ailes latérales 42 et 40 des volets respectifs 18 et 20 en contact avec les facettes d'extension respectives 50a et 52a de l'extension articulaire 32.

On voit ainsi que la fraction de la surface extérieure de chaque aile latérale qui serait masquée par l'extension articulaire 2 de la valve selon l'art antérieur n'est plus, avec l'invention, en vis-à-vis d'une surface matérielle, ce qui réduit considérablement le risque d'interposition d'un dépôt biologique entre cette fraction de surface extérieure et la surface interne latérale de l'extension articulaire. L'échancrure pratiquée dans chaque extension articulaire permet donc à une plus grande surface des ailes latérales de chaque volet d'être nettoyée par l'écoulement sanguin au cours du cycle cardiaque.

L'élimination de surfaces non biologiques en contact l'une avec l'autre dans l'espace de pivotement des volets élimine par conséquent ou, à tout le moins, réduit les risques de dépôt biologique de coagulation dans cette zone.

L'invention permet ainsi d'éliminer en pratique un risque vital de dysfonctionnement valvulaire entrainant une insuffisance circulatoire aigüe.

Il convient de noter que la somme des fractions des surfaces extérieures des deux ailes latérales de chaque volet, c'est-à-dire des facettes d'articulation 40a et 42a, qui sont agencées, en position ouverte du volet, contre les facettes d'extension respectives 52a et 50a de l'extension articulaire correspondante, correspond à une surface sensiblement inférieure à 5 % à la surface extérieure totale du volet.

Théoriquement, il n'y a pas de limite inférieure pour la surface des deux facettes d'articulation, dans la mesure où l'on souhaite qu'elle soit aussi faible que possible tout en assurant un guidage en rotation efficace du volet. Cependant, en pratique, une limite inférieure de 1 % est réalisable et la surface des deux facettes d'extension est ainsi, par exemple, égale à 1,4 % de la surface extérieure totale du volet.

On notera que pour réduire la surface en vis-à-vis des deux facettes d'articulation la largeur de la base de chaque extension articulaire peut être réduite par rapport à la largeur de son sommet, de façon à ce que l'extension visible sur la figure 3 ressemble plus à une forme de champignon qu'a celle d'un créneau.

Les flancs latéraux de l'extension seront ainsi concaves au lieu d'être sensiblement rectilignes comme sur la figure 3.

A titre de comparaison, la somme des fractions des surfaces extérieures des ailes latérales d'un volet de la valve de l'art antérieur décrite dans le brevet US 6 395 024 et qui sont en contact avec une partie de la surface interne de deux extensions articulaires correspondantes est au moins égale à 15 % de la surface extérieure totale du volet.

On comprend ainsi l'amélioration apportée par cet agencement de la présente invention aux valves de l'art antérieur et l'impact que cela peut avoir sur le traitement anticoagulant préventif à mettre en oeuvre pour éviter les risques d'interposition de matériels biologiques.

On notera que cet impact est d'autant plus élevé pour les valves à trois volets puisque celles-ci comportent six espaces de pivotement comparés à quatre pour les valves à deux volets.

Les figures 6 et 8 illustrent un volet 100 d'une valve cardiaque prothétique mécanique à trois volets selon l'art antérieur, respectivement en vue de dessus et en perspective.

Sur cette figure, le volet 100 comporte deux ailes latérales 102 et 104 qui sont raccordées respectivement à une partie centrale 106 par l'intermédiaire de zones de jonction 108, 110 formant chacune une zone convexe de très faible rayon de courbure. Cette zone de raccord s'apparente ainsi pour l'écoulement à une « arête » sur la surface extérieure du volet.

L'angle que forme chaque aile latérale avec la partie centrale du volet est constant.

Le Demandeur s'est aperçu que cette « arête » sur la surface extérieure du volet génère, dans l'écoulement, une singularité sous la forme d'une petite zone de re-circulation en aval, re-circulation qui se situe à proximité immédiate des facettes d'articulation et d'extension. Cette singularité augmente à cet endroit l'énergie cinétique des cellules sanguines et notamment des plaquettes, augmente leur temps de résidence sur les surfaces environnantes et augmente par conséquent le risque de formation de dépôts de coagulation.

En éliminant, comme on vient de le voir lors de la description faite en référence aux figures 3, 4a, 11 et 12, une grande partie de la surface latérale des extensions articulaires qui est adjacente à cette zone de recirculation, on réduit le risque de formation de dépôts biologiques de coagulation sur les facettes d'articulation et d'extension qui définissent entre elles les espaces de pivotement de la valve.

Néanmoins, les phénomènes évoqués ci-dessus de perturbation de l'écoulement sanguin persistent en raison de la présence des zones de jonction 108, 110 de chaque volet.

Pour éviter cela, il est prévu dans la configuration de la valve selon l'invention que les ailes latérales 40, 42 de chaque volet, par exemple du volet 18 représenté sur les figures 5 et 7, forment chacune avec la partie centrale 38 à laquelle elles sont reliées une zone de jonction 80, 82 de surface extérieure convexe, dont le rayon de courbure est suffisamment grand pour éviter la formation d'écoulements tourbillonnaires au voisinage de cette surface.

Plus particulièrement, si l'on considère la longueur de cette zone de jonction qui s'étend du bord d'attaque au bord de fuite (parallèlement à l'axe Z), ce rayon de courbure doit être suffisamment grand sur au moins une partie de sa longueur incluant le bord de fuite 46 du volet. Ainsi, le rayon de courbure à proximité du bord d'attaque 44 peut adopter une faible valeur et, sur une partie de la longueur de cette zone de jonction qui inclut le bord de fuite 46, une valeur plus élevée qui permet d'éviter à l'écoulement de se détacher de la surface extérieure du volet et de générer des perturbations locales.

Une faible valeur du rayon de courbure à proximité du bord d'attaque permet d'avoir recours à des moyens de soutien inférieurs de taille réduite et qui font donc peu d'obstruction à l'écoulement.

Cependant, la valeur du rayon de courbure augmente suivant la direction de l'écoulement antérograde le long du volet, c'est-à-dire en se dirigeant vers le bord de fuite de ce dernier.

Une forme de réalisation conforme à cet enseignement est, par exemple, illustrée sur les figures 5 et 7, où la surface extérieure convexe de la zone de jonction 80, 82 adopte la forme générale d'une portion de cône dont le sommet est situé vers l'amont de l'écoulement antérograde, c'est-à-dire du côté du bord d'attaque 44 du volet, et l'ouverture du cône est située au niveau du bord de fuite. Il convient de noter que le sommet du cône peut être placé plus ou moins près du bord d'attaque selon la forme souhaitée. Ainsi, le rayon de courbure augmente, par exemple, progressivement du bord d'attaque, ou à proximité de celui-ci, vers le bord de fuite. Les figures 13 et 14 illustrent respectivement les vues schématiques prises dans le plan du volet, du bord d'attaque 44 et du bord de fuite 46.

On notera que la surface intérieure de la zone de jonction 80, 82 a également la forme générale d'une portion de cône.

La valeur du rayon de courbure au bord d'attaque pour les valves implantées en position aortique est comprise entre 1 et 2 mm et est, par exemple, égale à 1,15 mm pour une valve de diamètre extérieur 19 mm et à 1,5 mm pour une valve de diamètre extérieur 31 mm.

La valeur du rayon de courbure au bord de fuite est d'au moins 2 mm, plus particulièrement, comprise entre 2 et 4 mm et est, par exemple, égale à 2,5 mm pour un diamètre de 19 mm et à 3,3 mm pour un diamètre de 31 mm.

Les valeurs correspondantes respectives des rayons de courbure sur la surface intérieure du volet sont de 0,5 et 0,6 mm pour le bord d'attaque et 1,5 et 1,8 mm pour le bord de fuite.

Pour les valves implantées en position mitrale, les valeurs de rayons de courbure au bord d'attaque sont comprises entre 1 et 2 mm et sont, par exemple, égales à 1,32 mm pour une valve de diamètre extérieur 25 mm et à 1,5 mm pour une valve de diamètre extérieur 33 mm. Elles sont d'au moins 2 mm au bord de fuite, plus particulièrement, comprises entre 2 et 4 mm et sont, par exemple, de 2,9 mm pour un diamètre de 25 mm et de 3,3 mm pour un diamètre de 33 mm.

Les valeurs correspondantes respectives des rayons de courbure sur la surface intérieure du volet sont de 0,52 et 0,6 mm pour le bord d'attaque et de 1,6 et 1,8 mm pour le bord de fuite.

On notera que si l'on augmente, au niveau du bord d'attaque, le rayon de courbure entre la partie centrale et l'aile latérale du volet, l'étendue de la surface de contact entre la surface d'extrémité supérieure du premier moyen de soutien inférieur et le bord d'attaque du volet, durant le mouvement de fermeture, augmente sensiblement, ce qui répartit encore mieux l'usure. La zone initiale de contact en début de fermeture se trouve alors sensiblement déplacée vers la pointe du premier moyen de soutien plutôt que vers sa base d'insertion.

Toutefois, un compromis doit être trouvé sur la valeur du rayon de courbure au niveau du bord d'attaque afin que les moyens de soutien inférieurs conservent une taille raisonnable vis-à-vis de l'écoulement.

A titre d'exemple, la valeur de l'angle au sommet du cône (mesurée au niveau du bord d'attaque) est de 50°, plus ou moins 5°.

Pour réduire davantage les singularités hydrodynamiques générées par les volets dans l'écoulement, on confère à la surface externe 45 de la partie centrale du volet 18 une forme, par exemple, sensiblement convexe suivant une direction prise en se déplaçant de l'aile latérale 40 à l'aile latérale opposée 42 (figure 15) au lieu d'une forme générale plane. Cette forme convexe ne concerne toutefois que la zone du volet proche du bord d'attaque, entre l'axe de rotation du volet et le bord d'attaque, la zone du volet située en aval de l'axe de rotation étant, quant à elle, plutôt concave. Ainsi, la course du bord d'attaque sur les premiers moyens de soutien inférieurs sera sensiblement plus courte, augmentant de ce fait la résistance à l'usure de la valve.

Selon une autre forme de réalisation (non représentée), la surface extérieure convexe de la zone de jonction entre la partie centrale du volet et chaque aile latérale adopte la forme générale d'une portion cylindrique et le rayon de courbure est donc constant.

Lorsque de tels volets équipent des valves implantées en position aortique, le rayon de courbure sur la surface extérieure des volets est d'au moins 2 mm, plus particulièrement compris entre 2 et 4mm et, par exemple, égal à 2,5 mm pour une valve de diamètre extérieur égal à 19 mm.II est compris en 2 et 4 mm et, par exemple, égal à 3,3 mm pour une valve de diamètre extérieur égal à 33 mm pour les valves implantées en position mitrale.

L'agencement de la zone de jonction en forme de portion de cylindre peut être utile dans certaines applications lorsque le rayon de courbure au voisinage du bord d'attaque du volet ne doit pas être le plus petit possible.

On notera que, quelle que soit la forme générale de la zone de jonction, afin d'éviter la formation d'écoulements tourbillonnaires au voisinage des zones d'articulation des volets (zones où les ailes latérales des volets coopèrent avec les extensions articulaires), la valeur minimale du rayon de courbure au niveau du bord de fuite est de 2 mm pour les valves destinées à être implantées en position aortique et de 3 mm pour les valves destinées à la position mitrale.

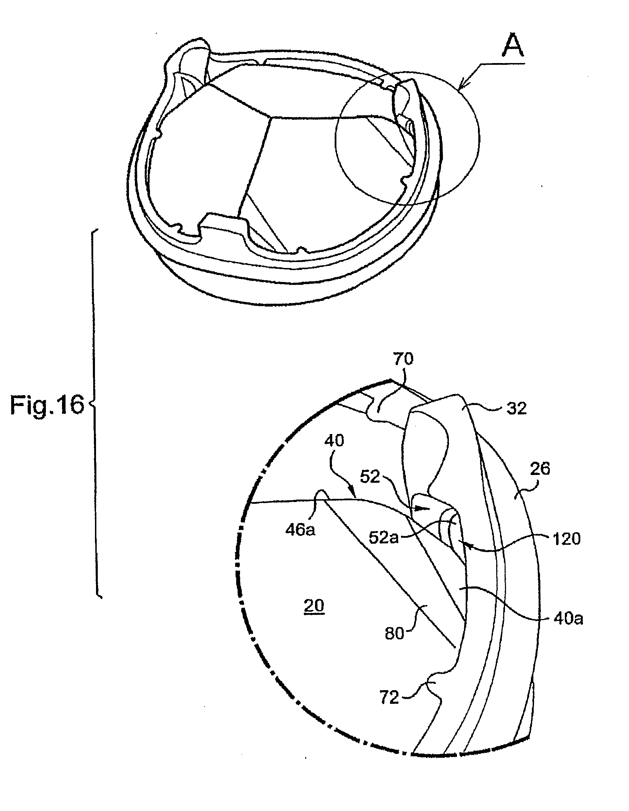

Lorsque les volets sont en position fermée (figures 2, 10, 16 et 17), chacune des facettes d'articulation de chaque volet (par exemple, la facette 40a sur la figure 16) et la facette d'extension correspondante (par exemple, la facette 52a sur la figure 16) de l'extension articulaire concernée (extension 32 sur la figure 16) définissent entre elles un espace libre 120, appelé espace de pivotement du volet et qui présente une forme géométrique tridimensionnelle se prêtant mal à une représentation figurative.

Cette forme est définie de façon théorique par le volume développé par le déplacement, dans l'espace, de la facette d'articulation 40a du volet au cours du mouvement d'ouverture/fermeture de ce volet.

Lorsque le volet est ouvert (figures 1, 3, 4a et 12), la facette d'articulation 40a est en contact avec la facette d'extension correspondante 52a et l'espace de pivotement 120 a disparu.

On notera que le volume de l'espace de pivotement est inférieur à 2/100^{e} du volume total déplacé par un volet lors de son passage de la position fermée à la position ouverte, volume qui est bien inférieur au volume de l'espace de pivotement d'un volet de l'art antérieur doté de l'extension articulaire 2 de la figure 3.

La valve comporte ainsi six espaces de pivotement 120 en position fermée (figures 2, 10 et 15).

Lorsque les zones de jonction 80, 82 des volets ont la forme d'une portion de cône ou d'un tronc de cône, on constate que la partie aval de ces zones (située du côté du bord de fuite 46) est abaissée par rapport à la partie de ces zones située en amont, c'est-à-dire du côté du bord d'attaque 44 (figures 12 et 14).

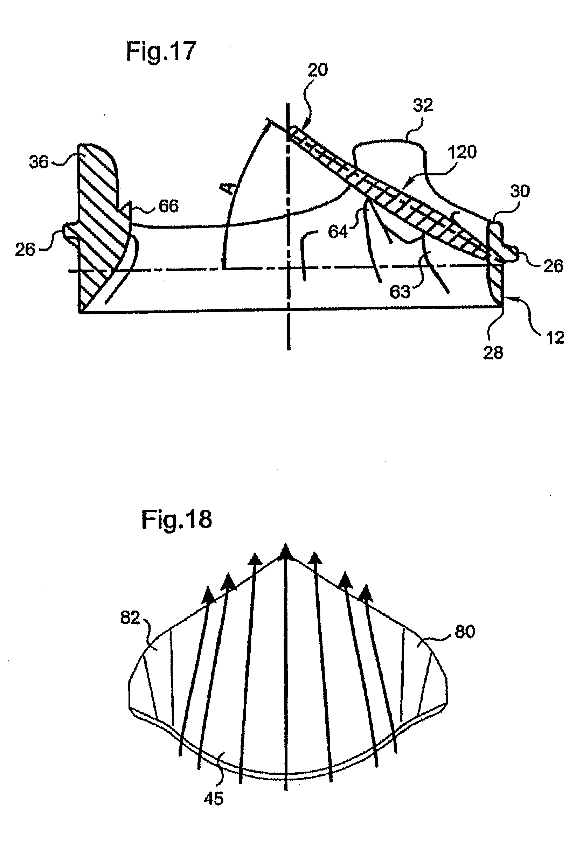

Ainsi, en position fermée des volets, la zone d'accolement entre les bords de fuite des volets est abaissée, par comparaison avec l'art antérieur, par rapport à un plan perpendiculaire à l'axe longitudinal X, tel que le plan contenant le bord d'attaque 28 du support annulaire 12 (figure 17).

L'angle A, appelé angle de fermeture et représenté sur la figure 17, est donc réduit grâce à l'invention.

Pour des valves destinées à être implantées en position aortique et en position mitrale, cet angle est compris entre 30 et 50° et une valeur d'angle de 35° convient particulièrement pour la position aortique. Pour les valves destinées à être implantées en position mitrale, un angle allant jusqu'à 50° peut se révéler avantageux. On notera toutefois qu'un angle de fermeture de 35° peut être adopté pour toutes les tailles de valves aortiques et mitrales.

Par ailleurs, du fait de l'abaissement des bords de fuite des volets par rapport à l'horizontale en position fermée du volet (figure 17), lorsque ce dernier est en appui sur les moyens de soutien inférieurs, l'espace de pivotement 120 (figure 16) devient plus évasé et plus accessible au rinçage rétrograde par le courant sanguin que dans les valves de l'art antérieur où cet espace est encaissé entre des parois moins évasées qui gênent davantage l'accès pour l'écoulement.

Ainsi, le risque que des dépôts de coagulation se forment et grossissent dans cet espace de pivotement est réduit grâce à l'agencement de l'invention.

Il convient de noter que les espaces de pivotement de la valve à trois volets rigides constituent des espaces critiques pour la résistance de la valve aux phénomènes de coagulation. L'agencement spécifique de cet espace selon l'invention a pour but de réduire le plus possible toute stase sur les parois adjacentes (volets et extensions articulaires), toute singularité dans la microstructure de l'écoulement à cet endroit et toute surface étrangère inutile à son voisinage immédiat.

Comme représenté plus particulièrement sur la figure 15 et déjà exposé ci-dessus, la surface extérieure 45 de la partie centrale 38 de chaque volet est de forme, par exemple, sensiblement convexe, ce qui augmente la surface centrale des volets exposée à l'écoulement antérograde lorsque la valve est en position ouverte. Conjuguée avec l'agencement de la zone de jonction à rayon de courbure augmenté entre la partie centrale et les ailes latérales des volets, cette convexité a pour but de répartir uniformément l'écoulement sur toute la surface extérieure des volets et notamment sur les facettes latérales dédiées au pivotement. Ceci est contraire à ce qui est réalisé par l'art antérieur décrit dans le brevet US 6 395 024 où la forme de la surface extérieure du volet tend à éloigner l'écoulement des ailes latérales en le dirigeant plus volontiers vers le centre du volet.

Ainsi, cette configuration permet de réduire les risques d'interposition biologique en cas d'implantation non exactement orthogonale par rapport à l'axe du flux, positionnement qui n'est pas rare en pratique du fait des modifications pathologiques locales que rencontrent souvent les chirurgiens lors de l'implantation d'une prothèse valvulaire.

Sur la figure 12 illustrant la valve selon l'invention en position ouverte, on constate que le passage interne 14 offert à l'écoulement est divisé en un orifice principal 14a et trois orifices secondaires 14b, 14c et 14d.

L'orifice principal est délimité par les surfaces intérieures des volets. La surface intérieure 47 de la partie centrale des volets a, de préférence dans sa partie amont, une forme générale concave suivant une direction prise en se déplaçant d'une aile latérale 40 à l'aile latérale opposée 42 (figure 15), ce qui positionne la partie amont de chaque volet incluant le bord d'attaque dans une zone de l'écoulement sanguin antérograde où les vitesses sont sensiblement plus lentes que vers le centre de la valve.

La partie amont est celle située entre le bord d'attaque et l'axe de rotation du volet.

Ainsi, l'écoulement antérograde rencontrant le bord d'attaque des volets est moins sujet à des perturbations qu'avec des volets dont la surface intérieure est de forme convexe dans le plan de la figure 15.

On notera que l'orifice principal est ainsi sensiblement élargi par rapport à l'art antérieur et la section de passage offerte à l'écoulement par cet orifice dans un plan perpendiculaire à l'axe X, notamment dans la partie de l'orifice définie par la partie amont des volets, est au moins égale à 75% de la surface interne délimitée par le support 12.

Chaque orifice secondaire 14b, 14c, 14d est, quant à lui, défini par l'espace offert à l'écoulement entre la surface extérieure d'un des trois volets et la portion de surface périphérique interne du support 12 qui sépare les extensions articulaires avec lesquelles le volet concerné coopère.

Lorsque la surface extérieure des volets a une forme générale sensiblement convexe, les orifices secondaires ont chacun une forme générale de croissant de lune.

Ces orifices secondaires constituent des orifices de rinçage des surfaces extérieures des volets et notamment de leurs ailes latérales.

On notera que la plus grande section de passage offerte à l'écoulement par chaque orifice secondaire 14b-d dans un plan perpendiculaire à l'axe X, est inférieure à 7% de la surface interne délimitée par le support 12.

Par ailleurs, la dimension de chaque orifice secondaire prise suivant une direction radiale passant par le centre du support 12, dans un plan perpendiculaire à l'axe X, est inférieure à 20% du rayon interne du support.

La figure 18 illustre la structure de l'écoulement sur la surface extérieure plane, voire concave 45 d'un volet en position ouverte.

C'est également le cas lorsque la surface extérieure du volet a la forme illustrée sur la figure 15 au voisinage du bord d'attaque, puis plutôt concave vers l'aval.

On constate que, de manière générale, l'écoulement converge vers la partie centrale du volet, ce qui favorise le nettoyage de cette partie au détriment des ailes latérales.

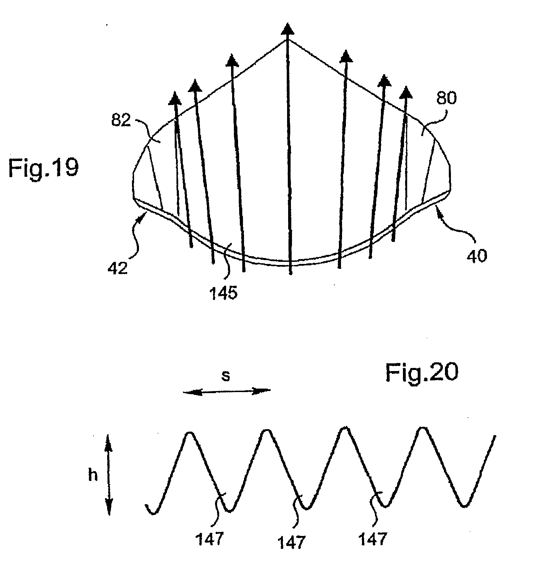

Dans la mesure où, comme évoqué précédemment, les parties de la valve situées près des espaces de pivotement de celle-ci constituent des zones critiques qui doivent être particulièrement bien nettoyées par l'écoulement, le Demandeur a modifié la structure de la surface extérieure des volets pour favoriser l'orientation de l'écoulement sanguin vers les ailes latérales des volets tel qu'illustré sur la figure 19.

La surface extérieure modifiée 145 est ainsi pourvue d'une pluralité de rainures 147 représentées à titre d'exemple à la figure 20 avec une section transversale en V et qui sont orientées de manière à canaliser le flux sanguin de façon contrôlée.

Les rainures peuvent être orientées différemment selon les zones de la surface extérieure du volet où elles sont agencées : les rainures agencées près du centre du volet sont orientées axialement suivant l'axe de symétrie Z du volet, tandis que les rainures agencées à proximité des ailes latérales 40, 42 ont une orientation axiale qui forme avec l'axe Z un angle, par exemple, compris entre 5° et 7.

Cet angle peut être de plus en plus prononcé au fur et à mesure que les rainures sont proches des ailes.

Un tel agencement répartit l'écoulement sur une plus grande surface du volet et favorise ainsi le nettoyage des ailes latérales.

On notera que d'autres formes possibles de sections transversales des rainures sont envisageables : formes arrondies en U, formes rectangulaires, formes trapézoïdales, ailettes en forme de L ...

Ces rainures ont une hauteur h qui correspond sensiblement à l'épaisseur de la couche limite de l'écoulement sanguin sur le volet et qui est, par exemple, de l'ordre de 0,01 mm. De façon générale, l'épaisseur de la couche limite peut être obtenue à partir des dimensions d'un volet, en appliquant un facteur d'échelle de 1/ (nombre de Reynolds) ½.

On notera que l'espacement s (largeur de rainure) sur la figure 20 peut être augmenté si nécessaire.

Afin de réduire le risque de contamination des rainures, un espacement minimum s de 5 mm est efficace.

On notera également que la distance séparant deux rainures consécutives est ajustée en fonction des risques de contamination des rainures.

Par ailleurs, les rainures aménagées sur tout ou partie de la surface extérieure des volets contribuent à épaissir et à stabiliser la couche limite de l'écoulement, réduisant ainsi le frottement turbulent et la traînée de frottement résultante générés par la rencontre de l'écoulement et de la surface extérieure des volets.

Ces rainures sont obtenues de façon connue, par exemple, par moulage lorsque les volets sont réalisés en polymères biocompatibles, ou par un dépôt de diamant isotrope sur quelques microns d'épaisseur si les volets sont fabriqués à partir d'un autre matériau, ou encore par micro-usinage.

Il convient de noter que la surface intérieure des volets peut également être rainurée pour favoriser une répartition différente de l'écoulement.

La nervure périphérique 26 prévue pour la fixation d'un anneau de suture (non représenté) est par exemple configurée de façon spécifique afin que sa forme générale, que l'on aperçoit sur les figures 1 à 3, reproduise le profil d'une courbe sensiblement sinusoïdale.

Ainsi, les sommets de la courbe sinusoïdale (la courbure de ces sommets a été volontairement exagérée pour une plus grande visibilité) sont respectivement agencés au droit de chacune des extensions articulaires 32, 34, 36 (sommet 26a au droit de l'extension 34) du support et les creux sont respectivement agencés entre deux extensions articulaires consécutives : le creux 26b est agencé entre les extensions 34 et 36, tandis que le creux 26c est agencé entre les extensions 32 et 34.

D'une certaine manière, on peut dire que le profil de la nervure 26 suit de façon générale le contour du bord de fuite 30 du support 12.

Pour fabriquer la valve à volets rigides selon l'invention, différents matériaux peuvent être utilisés.

Pour le support annulaire, on choisit, par exemple, un métal biocompatible tel que le titane ou le stellite.

On peut également utiliser du carbone massif, voire un revêtement de carbone sur du graphite.

Les volets quant à eux peuvent être réalisés à partir d'un matériau biocompatible, par exemple du carbone monolithique, ou en graphite avec un revêtement de carbone pyrolytique.

Les volets peuvent aussi être réalisés dans un polymère de synthèse biocompatible et qui présente des propriétés de résistance à l'usure comparables à celles du carbone pyrolytique.

Ainsi, un matériau tel que le "Peek" (acronyme pour "Polyetheretherkétone") possède une faible densité de l'ordre de 1,2 et convient particulièrement pour fabriquer les volets.

Ce matériau est renforcé en carbone afin d'augmenter la résistance à l'usure des volets.

Un tel matériau est fourni, par exemple, par la société Ensinger GmbH & Co., D-93413 Allemagne. Un tel matériau adapté à un usage médical est également disponible auprès de la société britannique Invibio Ltd.

On notera que la valve selon l'invention peut être réalisée en titane pour le support annulaire 12 et en "peek" pour les volets, ce qui procure un couple de matériaux parfaitement adapté aux frottements et usures rencontrés sur ce type de valves.

Par ailleurs, on peut également utiliser le "Peek" comme matériau pour fabriquer les volets et le carbone pyrolitique pour le support, voire le carbone pyrolitique pour les volets et le support.

Un tel choix de matériaux peut d'ailleurs être retenu pour d'autres types de valves cardiaques à volets rigides indépendants de l'invention.

## Revendications

1. Valve cardiaque prothétique mécanique, comprenant:
- un support annulaire (12) comportant une surface périphérique (16) interne centrée autour d'un axe longitudinal (X),
- au moins deux volets mobiles (18, 20, 22) qui sont agencés de façon articulée sur la surface périphérique interne du support de manière à pouvoir effectuer chacun un mouvement de rotation autour d'un axe de rotation de volet perpendiculaire à l'axe longitudinal (X), pour passer d'une position ouverte de la valve, dans laquelle les volets ouverts délimitent entre eux un orifice principal (14a) centré sur l'axe longitudinal et à travers lequel le sang s'écoule axialement, à une position fermée de la valve, dans laquelle les volets fermés empêchent le sang de refluer à travers l'orifice principal,
le support annulaire (12) comportant un bord (30) placé du côté aval de l'écoulement antérograde, appelé bord aval, et plusieurs extensions articulaires (32, 34, 36) qui s'étendent axialement à partir du bord aval et dont le nombre correspond à celui des volets, chaque volet comportant une partie centrale (38) encadrée de façon symétrique par deux ailes latérales (40, 42) qui sont inclinées par rapport à cette partie centrale, ces deux ailes coopérant respectivement, pour permettre la rotation du volet, avec les surfaces internes de deux extensions articulaires par l'intermédiaire d'une portion dite terminale (40a, 42a) de chaque aile, chaque volet comportant à sa périphérie, d'une part, un bord d'attaque (44) qui est disposé du côté amont de l'écoulement antérograde de sang et coopère avec la surface interne (16) du support annulaire en position fermée du volet et, d'autre part, un bord de fuite (46) disposé du côté aval de l'écoulement antérograde, et qui comprend deux portions symétriques (46a, 46b) qui s'étendent respectivement depuis les portions terminales des ailes latérales (40, 42) jusqu'à une zone d'extrémité aval (48) où elles se rejoignent pour former une pointe, chaque portion terminale de chaque aile latérale de chacun des volets comportant un bord latéral reliant le bord d'attaque et le bord de fuite, chaque volet comportant une surface extérieure tournée en position ouverte vers la surface périphérique (16) interne du support annulaire (12) et une surface intérieure tournée en position ouverte vers l'orifice principal (14a) de la valve et qui sont reliées l'une à l'autre par le bord d'attaque (44), le bord de fuite (46) et les deux bords latéraux respectifs des deux portions terminales (40a, 42a) des deux ailes (40, 42), la surface extérieure de chaque volet qui inclut la surface extérieure des ailes latérales ayant une forme générale convexe suivant une direction prise en se déplaçant d'une portion terminale (40a) d'une aile latérale (40) à la portion terminale (42a) de l'aile latérale opposée, la surface intérieure ayant une forme générale concave suivant ladite direction, **caractérisée en ce que** chaque portion terminale possède une surface extérieure, appelée facette d'articulation, qui est une fraction de la surface extérieure du volet et qui vient en appui, lorsque le volet est ouvert, contre une portion de la surface interne de l'extension articulaire correspondante, appelée facette d'extension, les deux facettes d'articulation de chaque volet totalisant à elles deux une surface sensiblement inférieure à 5 % de la surface extérieure du volet.

2. Valve selon la revendication 1, **caractérisée en ce que** chaque aile latérale (40, 42) de chacun des volets est reliée à la partie centrale (38) du volet par une zone de jonction (80, 82) dont la surface extérieure est convexe et qui, sur au moins une partie de sa longueur incluant la partie de la zone située vers l'aval de l'écoulement antérograde, possède un rayon de courbure suffisamment grand pour éviter la formation d'écoulements tourbillonnaires au voisinage de cette surface de jonction en position ouverte des volets, ladite longueur de la zone de jonction s'étendant du bord d'attaque (44) au bord de fuite (46).

3. Valve selon la revendication 2, **caractérisée en ce que** le rayon de courbure de la partie de la zone de jonction située du côté aval de l'écoulement est d'au moins 2 mm pour une valve destinée à être implantée en position aortique et d'au moins 3 mm pour une valve destinée à être implantée en position mitrale.

4. Valve selon l'une des revendications 1 à 3, **caractérisée en ce que** chaque aile latérale (40, 42) de chacun des volets est reliée à la partie centrale (38) du volet par une zone de jonction (80, 82) dont la surface extérieure est convexe et a la forme générale soit d'une portion de cône dont le sommet est situé vers l'amont de l'écoulement antérograde, soit d'une portion de cylindre.

5. Valve selon l'une des revendications 1 à 4, **caractérisée en ce que** chacune des facettes d'articulation (40a, 42a) d'un volet et la facette d'extension correspondante (52a, 50a) de l'extension articulaire concernée définissent entre elles, lorsque le volet est en position fermée, un espace (120) dit de pivotement du volet, cet espace disparaissant lorsque la facette d'articulation du volet vient, en position ouverte, en appui contre la facette d'extension correspondante.

6. Valve selon la revendication 5, **caractérisée en ce que** le volume de l'espace de pivotement (120) est inférieur à 2/100e du volume déplacé par le volet lors de son passage de la position fermée à la position ouverte.

7. Valve selon l'une des revendications 1 à 6, **caractérisée en ce que**, lorsque la valve est en position ouverte, chaque volet définit, entre sa surface extérieure (45) et la portion de surface périphérique interne (16) du support annulaire (12) qui sépare les deux extensions articulaires avec lesquelles le volet coopère, un orifice secondaire qui présente au moins l'une des caractéristiques suivantes :
- chaque orifice secondaire (14b, 14c, 14d) a une forme générale de croissant de lune,
- la dimension de l'orifice secondaire, prise suivant une direction radiale, en projection dans un plan perpendiculaire à l'axe longitudinal du support annulaire, est inférieure à 20 % du rayon interne du support annulaire,
- chaque orifice secondaire (14b, 14c, 14d) présente, dans un plan perpendiculaire à l'axe longitudinal du support annulaire, une section de passage offerte à l'écoulement qui est inférieure à 7 % de la surface interne délimitée par le support annulaire dans ce même plan.

8. Valve selon l'une des revendications 1 à 7, **caractérisée en ce que** les extensions articulaires (32, 34, 36) sont dépourvues chacune de toute ouverture traversante.

9. Valve selon l'une des revendications 1 à 8, **caractérisée en ce que** le support annulaire (12) comporte sur sa surface périphérique interne (16), à proximité du bord aval (30) et pour chaque volet (18), deux butées (70, 71) provoquant le pivotement du volet dans sa position ouverte lorsque la pression de l'écoulement de sang s'exerce sur la face interne de ce volet.

10. Valve selon l'une des revendications 1 à 9, **caractérisée en ce que** le support annulaire comporte sur sa surface périphérique interne (16), pour chaque volet (18), deux moyens de soutien (60, 62) du volet en position fermée, lesdits moyens de soutien de chaque volet étant agencés entre les deux extensions articulaires (32, 36) avec lesquelles coopèrent respectivement les ailes latérales (42, 40) du volet.

11. Valve selon les revendications 9 et 10, **caractérisée en ce que**, en projection dans un plan perpendiculaire à l'axe longitudinal (X) du support annulaire, chaque butée (70, 71) est espacée angulairement du moyen de soutien (70, 71) le plus proche d'une distance correspondant sensiblement à au moins la moitié de la largeur dudit moyen de soutien, la largeur étant mesurée, dans le plan considéré, suivant une direction tangentielle par rapport au support annulaire.

12. Valve selon la revendication 11, **caractérisée en ce que**, pour chaque volet (18), les butées (70, 71) sont agencées entre les moyens de soutien (60, 62) du volet.

13. Valve selon l'une des revendications 1 à 12, **caractérisée en ce que** chaque volet, d'une part, en position fermée, forme avec un plan perpendiculaire à l'axe longitudinal (X) du support annulaire un angle de fermeture compris entre 30° et 50° et plus particulièrement est compris entre 40° et 50° pour les valves destinées à être implantées en position mitrale, et, d'autre part, en position ouverte, est sensiblement parallèle à la direction de l'écoulement.

14. Valve selon l'une des revendications 1 à 13, **caractérisée en ce que** le support annulaire comporte sur sa surface périphérique extérieure, pour les valves destinées à être implantées en position aortique, une nervure périphérique (36) pour la fixation d'un anneau de suture, la nervure étant configurée de manière à ce que sa forme générale reproduise le profil d'une courbe sensiblement sinusoïdale ayant un sommet (26a) agencé au droit de chaque extension articulaire eut un creux (26b, 26c) entre deux extensions articulaires consécutives.

## Patentansprüche

1. Mechanische prothetische Herzklappe, mit:
- einem ringförmigen Stützelement (12) einschliesslich einer inneren um eine Längsachse (X) zentrierten peripheren Oberfläche (16),
- mindestens zwei beweglichen Segeln (18, 20, 22), welche derart auf eine gelenkige Weise auf der inneren Oberfläche des Stützelements angebracht sind, dass jede in der Lage ist, eine Drehbewegung um eine zur Längsachse (X) senkrechte Segelrotationsachse auszuführen, um so zu wechseln von einer offenen Stellung der Klappe, worin die offenen Segel eine auf der Längsachse zentrierte Hauptöffnung (14a) dazwischen abgrenzen und durch welche Blut axial fliesst, zu einer geschlossenen Stellung der Klappe, worin die geschlossenen Segel das Blut am Rückfluss durch die Hauptöffnung hindern,
worin das ringförmige Stützelement (12) einen Rand (30) auf der stromabwärtigen Seite des anterograden Abflusses umfasst, einen sogenannten stromabwärts gerichteten Rand, und mehrere Gelenkerweiterungen (32, 34, 36), welche sich axial vom stromabwärtigen Rand erstrecken und deren Anzahl derjenigen der Segel entspricht, worin jedes Segel einen zentralen Teil (38) aufweist, der auf eine symmetrische Weise durch zwei in Bezug auf diesen Teil geneigte Seitenflügel (40, 42) eingegrenzt ist, worin diese zwei Flügel jeweils zusammenwirken, um das Drehen des Segels mit den inneren Oberflächen von zwei Gelenkerweiterungen durch ein sogenanntes Endteil (40a, 42a) jedes Flügels zu ermöglichen, wobei jedes Segel an seiner Aussenseite einerseits eine Führungskante (44) aufweist, die auf der stromaufwärtige Seite des anterograden Blutabflusses situiert ist und bei geschlossener Position des Segels mit der Innenoberfläche (16) des ringförmigen Stützelements zusammenarbeitet, und, anderseits, eine Hinterkante (46), die auf der stromaufwärtige Seite des anterograden Abflusses angebracht ist und zwei symmetrische Teile (46a, 46b) aufweist, welche sich jeweils vom Endteil der Seitenflügel (40, 42) bis in eine stromabwärtige Endzone (48) erstrecken, wo sie sich zu einem Punkt zusammenfügen, wobei jedes Endteil jedes Seitenflügels von jedem Segel eine die Führungskante und die Hinterkante verbindende Seitenkante aufweist, wobei jedes Segel eine Aussenoberfläche aufweist, welche in der offenen Position zur inneren peripheren Oberfläche (16) des ringförmigen Stützelements (12) zugewendet ist, und eine Innenoberfläche aufweist, welche in der offenen Position zur Hauptöffnung (14a) der Klappe zugewendet ist und welche miteinander durch die Führungskante (44), die Hinterkante (46) und die beiden jeweiligen Aussenseiten der beiden Endteile (40a, 42a) der beiden Flügel (40, 42) verbunden sind, wobei die die Aussenoberfläche der Seitenflügel beinhaltende Aussenoberfläche des jeweiligen Segels eine allgemein konvexe Form aufweist, entlang einer Richtung der Bewegung eines Endteils (40a) eines Seitenflügels (40) zum Endteil (42a) des gegenüberliegenden Flügels, wobei die Innenoberfläche eine allgemein konkave Form entlang der besagten Richtung aufweist, **dadurch gekennzeichnet, dass** jedes Endteil eine Gelenkaspekt genannte Aussenoberfläche aufweist, die ein Bruchteil der Aussenoberfläche des Segels darstellt und die, bei offenem Segel, gegen einen Teil der Innenoberfläche des entsprechenden, Erweiterungsaspekt genannten Gelenkaspekts zu ruhen kommt, worin die beiden Gelenkaspekte jedes Segels zusammen eine Gesamtoberfläche haben, welche deutlich weniger als 5% der Aussenoberfläche des Segels ausmacht.

2. Klappe gemäss Anspruch 1, **dadurch gekennzeichnet, dass** jeder Seitenflügel (40, 42) jedes der Segel mit dem zentralen Teil (38) des Segel mittels einer Verbindungszone (80, 82) verbunden ist, deren Aussenoberfläche konvex ist und welche zumindest auf Teil ihrer Länge einschliesslich des sich stromabwärtig des anterograden Abflusses befindenden Teils einen genügend weiten Kurvenradius aufweist, um die Bildung von Wirbelströmungen in der Nähe dieser Verbindungszone bei offener Position der Segeln zu verhindern, wobei die besagte Länge der Verbindungszone sich von der Führungskante (44) zur Hinterkante (46) erstreckt.

3. Klappe gemäss Anspruch 2, **dadurch gekennzeichnet, dass** der Kurvenradius des sich stromaufwärtig des anterograden Abflusses befindenden Teils der Verbindungszone mindestens 2mm für eine in eine aortische Stellung zu implantierende Klappe und mindestens 3mm für eine in eine mitrale Stellung zu implantierende Klappe beträgt.

4. Klappe gemäss einem der Ansprüche 1 bis 3, **dadurch gekennzeichnet, dass** jeder Seitenflügel (40, 42) jedes der Segel mit dem zentralen Teil (38) des Segels mittels einer Verbindungszone (80, 82) verbunden ist, deren Aussenoberfläche konvex ist und allgemein die Form entweder eines Kegelausschnitts mit einem sich stromabwärtig des anterograden Abflusses befindende Apex oder einer Portion eines Zylinders aufweist.

5. Klappe gemäss einem der Ansprüche 1 bis 4, **dadurch gekennzeichnet, dass** jede der Gelenkaspekte (40a, 42a) eines Segels und die entsprechenden Erweiterungsaspekte (52a, 50a) der jeweiligen Gelenkerweiterung beim Segel in geschlossener Position untereinander einen Raum (120) definieren, genannt Segeldrehraum, wobei dieser Raum verschwindet, wenn der Gelenkaspekt des Segels bei offener Stellung gegen den entsprechenden Erweiterungsaspekt zur ruhen kommt.

6. Klappe gemäss Anspruch 5, **dadurch gekennzeichnet, dass** das Volumen des Drehraums (120) weniger als 2 Hundertstel des durch das Segel verdrängten Volumens ist, wenn es von der geschlossenen zur offenen Stellung wechselt.

7. Klappe gemäss einem der Ansprüche 1 bis 6, **dadurch gekennzeichnet, dass** mit der Kappe in offener Position jedes Segel zwischen seiner Aussenoberfläche (45) und dem Teil der inneren peripheren Oberfläche (16) des ringförmigen Stützelements (12), welches die zwei Gelenkerweiterungen trennt, mit denen das Segel zusammenarbeitet, eine sekundäre Öffnung definiert, welche zumindest eines der folgenden Merkmale aufweist:
- jede sekundäre Öffnung (14b, 14c, 14d) hat die allgemeine Form eines Halbmondes,
- die Grösse der sekundären Öffnung, gemessen entlang einer radialen Richtung in Projektion in einer Ebene senkrecht zur Längsachse des ringförmigen Stützelements, ist geringer als 20% des Innenradius der Ringstütze,
- jede sekundäre Öffnung (14b, 14c, 14d) besitzt in einer Ebene senkrecht zur Längsachse des ringförmigen Stützelements einen zum Abfluss offenen Durchflussabschnitt, der weniger als 7% der durch das ringförmige Stützelement in derselben Ebene abgegrenzte Innenoberfläche ist.

8. Klappe gemäss einem der Ansprüche 1 bis 7, **dadurch gekennzeichnet, dass** die Gelenkerweiterungen (32, 34, 36) jeweils keine durchquerende Öffnung haben.

9. Klappe gemäss einem der Ansprüche 1 bis 8, **dadurch gekennzeichnet, dass** das ringförmige Stützelement (12) auf seiner inneren peripheren Oberfläche (16), in der Nähe des stromaufwärtigen Rands (30) und für jedes der Segel (18), zwei Anschläge (70, 71) aufweist, welche das Segel dazu veranlassen, in die geöffnete Stellung zu schwenken, wenn der Druck des ausfliessenden Blutes auf der inneren Seite dieses Segels ausgeübt wird.

10. Klappe gemäss einem der Ansprüche 1 bis 9, **dadurch gekennzeichnet, dass** das ringförmige Stützelement auf seiner inneren peripheren Oberfläche (16) für jedes Segel (18) zwei Stützmittel (60, 62) des Segels in geschlossener Position aufweist, worin die besagte Stützmittel eines jeden Segels zwischen den zwei Gelenkerweiterungen (32, 36) angebracht sind, mit denen die Seitenflügel (42, 40) der jeweiligen Segel zusammenarbeiten.

11. Klappe gemäss den Ansprüchen 9 und 10, **dadurch gekennzeichnet, dass** bei Projektion in einer Ebene senkrecht zur Längsachse (X) des ringförmigen Stützelements, jeder Anschlag (70, 71) vom am nächsten gelegenen Stützmittel (60, 62) um einen Abstand winkelversetzt ist, der ungefähr mindestens der Hälfte der Breite des besagten Stützmittels entspricht, wobei die Breite in der betrachteten Ebene entlang einer tangentialen Richtung in Bezug auf das ringförmige Stützelement gemessen wird.

12. Klappe gemäss Anspruch 11, **dadurch gekennzeichnet, dass** für jedes Segel (18) die Anschläge (70, 71) zwischen den Segelstüztmitteln (60, 62) angebracht sind.

13. Klappe gemäss einem der Ansprüche 1 bis 12, **dadurch gekennzeichnet, dass** jedes Segel einerseits bei geschlossener Stellung mit einer Ebene senkrecht zur Längsachse (X) des ringförmigen Stützelements einen Schliesswinkel bildet zwischen 30° und 50° und vorzugsweise zwischen 40° und 50° für Klappen zur Implantierung in einer mitralen Stellung, und andererseits bei offener Stellung ungefähr parallel zur Richtung des Abflusses ist.

14. Klappe gemäss einem der Ansprüche 1 bis 13, **dadurch gekennzeichnet, dass** das ringförmige Stützelement auf seiner äusseren peripheren Fläche für zur Implantierung in einer aortischen Stellung vorgesehene Klappen eine periphere Kerbe (36) zur Befestigung eines Nahtringes aufweist, wobei die Kerbe so angelegt ist, das ihre allgemeine Form das Profil einer ungefähren Sinuskurve wiedergibt, mit einem direkt im Weg jeder Gelenkerweiterung angeordneten Maximum (26a) und einem zwischen zwei aufeinanderfolgenden Gelenkerweiterungen angeordneten Minimum (26b, 26c).

## Claims

1. Mechanical prosthetic heart valve, comprising:
- an annular support (12) comprising an inner peripheral surface (16) centred around a longitudinal axis (X),
- at least two mobile shutters (18, 20, 22) that are arranged in an articulated manner on the inner peripheral surface of the support so as to be capable of performing each a rotation movement around a shutter rotation axis perpendicular to the longitudinal axis (X) to switch from an open position of the valve, wherein the open shutters delimit between them a main orifice (14a) centred on the longitudinal axis and through which blood flows axially, to a closed position of the valve, wherein the closed shutters prevent the blood from flowing back through the main orifice,
wherein the annular support (12) comprises a rim (30) placed on the downstream side of the anterograde outflow, called downstream rim, and several articular extension (32, 34, 36) extending axially from the downstream rim and whose number corresponds to that of the shutters, wherein each shutter comprises a central part (38) framed in a symmetrical fashion by two lateral wings (40, 42) that are inclined relative to this central part, wherein these two wings cooperate respectively, to enable the shutter to rotate, with the inner surfaces of two articular extensions through a so-called end portion (40a, 42a) of each wing, wherein each shutter comprises at its periphery on the one hand a leading edge (44) that is situated on the upstream side of the anterograde blood outflow and working together with the inner surface (16) of the annular support when the shutter is in closed position and, on the other hand, a trailing edge (46) that is situated on the downstream side of the anterograde outflow and comprising two symmetric portions (46a, 46b) extending respectively from the end portion of the lateral wings (40, 42) until a downstream end zone (48) where they join up to form a point, wherein each end portion of each lateral wing of each of the shutters comprises a lateral edge connecting the leading edge and the trailing edge, wherein each shutter comprises an outer surface that in the open position is turned towards the inner peripheral surface (16) of the annular support (12) and an inner surface that in the open position is turned towards the main orifice (14a) of the valve and which are connected to one another by the leading edge (44), the trailing edge (46) and the two respective lateral sides of the two end portions (40a, 42a) of the two wings (40, 42), wherein the outer surface of each shutter which includes the outer surface of the lateral wings has a generally convex shape following a direction taken by moving from a end portion (40a) of one lateral wing (40) to the end portion (42a) of the opposite lateral wing, wherein the inner surface has a generally concave shape following said direction, **characterized in that** each end portion has an outer surface, called articulation facet, which is a fraction of the shutter's outer surface and which, when the shutter is open, comes to rest against a portion of the inner surface of the corresponding articular extension, called extension facet, wherein the two articulation facets of each shutter together have a total surface considerably less than 5% of the shutter's outer surface.

2. Valve according to claim 1, **characterized in that** each lateral wing (40, 42) of each of the shutters is connected to the central part (38) of the shutter by a junction zone (80, 82) whose outer surface is convex and which, on at least part of its length including the part of the zone located towards downstream of the anterograde outflow, has a curve radius sufficiently wide to avoid the formation of vortex flows close to this junction surface when the shutters are in open position, wherein said length of the junction zone extends from the leading edge (44) to the trailing edge (46).

3. Valve according to claim 2, **characterized in that** the curve radius of the part of the junction zone located upstream of the outflow is of at least 2mm for a valve designed to be implanted in an aortic position and of at least 3mm for a valve designed to be implanted in a mitral position.

4. Valve according to one of the claims 1 to 3, **characterized in that** each lateral wing (40, 42) of each of the shutters is connected to the central part (38) of the shutter by a junction zone (80, 82) whose outer surface is convex and has the general shape either of a section of a cone whose apex is situated towards the downstream of the anterograde outflow or of a section of a cylinder.

5. Valve according to one of the claims 1 to 4, **characterized in that** each of the articulation facets (40a, 42a) of a shutter and the corresponding extension facet (52a, 50a) of the relevant articular extension define between them, when the shutter is in a closed position, a space (120) called shutter-pivoting space, wherein this space disappears when the articulation facet of the shutter, when in open position, comes to rest against the corresponding extension facet.

6. Valve according to claim 5, **characterized in that** the volume of the pivoting space (120) is less than 2/100^{th} of the volume displaced by the shutter when it switches from the closed position to the open position.

7. Valve according to one of the claims 1 to 6, **characterized in that**, when the valve is in an open position, each shutter defines between its outer surface (45) and the portion of the inner peripheral surface (16) of the annular support (12) separating the two articular extensions with which the shutter works, a secondary orifice that has at least one of the following characteristics:
- each secondary orifice (14b, 14c, 14d) has the general shape of a crescent moon,
- the size of the secondary orifice, taken along a radial direction, when projected in a plane perpendicular to the longitudinal axis of the annular support, is less than 20% of the inner radius of the annular support,
- each secondary orifice (14b, 14c, 14d) has, in a plane perpendicular to the longitudinal axis of the annular support, a flow section open to the outflow that is less than 7% of the inner surface delineated by the annular support in this same plane.

8. Valve according to one of the claims 1 to 7, **characterized in that** the articular extensions (32, 34, 36) are each without any traversing opening.

9. Valve according to one of the claims 1 to 8, **characterized in that** the annular support (12) comprises on its inner peripheral surface (16), close to the upstream rim (30) and for each shutter (18), two stops (70, 71) causing the shutter to pivot into its opened position when the pressure of the outflowing blood is exerted on the inner face of this shutter.

10. Valve according to one of the claims 1 to 9, **characterized in that** the annular support comprises on its inner peripheral surface (16), for each shutter (18), two support means (60, 62) of the shutter in closed position, wherein said support means of each shutter are arranged between the two articular extensions (32, 36) with which the lateral wings (42, 40) of the shutter respectively work.

11. Valve according to the claims 9 and 10, **characterized in that**, when projected in a plane perpendicular to the longitudinal axis (X) of the annular support, each stop (70, 71) is spaced in an angular fashion from the closest support means (60, 62) by a distance corresponding roughly to at least half of the width of said support means, wherein the width is measured, in the plane under consideration, along a tangential direction relative to the annular support.

12. Valve according to claim 11, **characterized in that**, for each shutter (18), the stops (70, 71) are arranged between the shutter's support means (60, 62).

13. Valve according to one of the preceding claims 1 to 12, **characterized in that** each shutter, on the one hand, when in closed position, forms with a plane perpendicular to the longitudinal axis (X) of the annular support an angle of closing comprised between 30° and 50° and preferably comprised between 40° and 50° for valves designed to be implanted in a mitral position and, on the other hand, when in open position, is roughly parallel to the direction of outflow.

14. Valve according to one of the claims 1 to 13, **characterized in that** the annular support comprises on its outer peripheral surface, for valves designed to be implanted in an aortic position, a peripheral groove (36) for fastening a suture ring, wherein the groove is configured such that its general shape reproduces the profile of a roughly sinusoidal curve having a peak (26a) arranged directly in the way of each articular extension and a trough (26b, 26c) between two consecutive articular extensions.
